(19) Europäisches Patentamt
European Patent Office
Office européen des brevets

(11) **EP 4 276 105 A1**

(12) **EUROPEAN PATENT APPLICATION**
published in accordance with Art. 153(4) EPC

(43) Date of publication:
**15.11.2023 Bulletin 2023/46**

(21) Application number: **21917328.3**

(22) Date of filing: **30.12.2021**

(51) International Patent Classification (IPC):
**C07K 7/08** (2006.01)    **C07K 7/06** (2006.01)
**A61K 38/10** (2006.01)    **A61K 38/08** (2019.01)
**A61P 1/04** (2006.01)    **A61P 17/02** (2006.01)

(52) Cooperative Patent Classification (CPC):
**A23L 33/18; A61K 8/64; A61K 38/07; A61K 38/08;
A61K 38/10; A61P 1/00; A61P 1/02; A61P 1/04;
A61P 1/14; A61P 7/04; A61P 7/10; A61P 17/00;
A61P 17/02; A61P 29/00; A61P 35/00;**    (Cont.)

(86) International application number:
**PCT/CN2021/142838**

(87) International publication number:
**WO 2022/148289 (14.07.2022 Gazette 2022/28)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **11.01.2021   CN 202110029899**

(71) Applicant: **Sichuan Gooddoctor Panxi
Pharmaceutical Co. Ltd
Sichuan 615000 (CN)**

(72) Inventor: **GENG, Funeng
Xichang City,  Liangshan Yi Sichuan 615000 (CN)**

(74) Representative: **Mewburn Ellis LLP
Aurora Building
Counterslip
Bristol BS1 6BX (GB)**

Remarks:
The complete document including Reference
Table(s) and the Sequence Listing(s) can be
downloaded from the EPO website

(54) **POLYPEPTIDE FOR REPAIRING SKIN WOUND OR MUCOSAL INJURY, AND APPLICATION THEREOF**

(57)    A polypeptide for repairing a skin wound or a mucosal injury, having obvious effects of alleviating the pathological development of acute and chronic gastrointestinal diseases, promoting repair of a skin wound or a mucosal injury, treating tumors, etc.; and use of the polypeptide in repairing a skin wound or a mucosal injury. By blocking or activating FGF/FGFR1 signaling, the polypeptide capable of binding to an FGFR1 receptor is capable of inhibiting or promoting cell proliferation, vascular hyperplasia, collagen formation, etc.

EP 4 276 105 A1

(52) Cooperative Patent Classification (CPC): (Cont.)
**A61Q 17/00; A61Q 19/00; C07K 5/1005;
C07K 5/1021; C07K 7/06; C07K 7/08**

**Description**

**Technical Field**

The present invention relates to a polypeptide for repairing skin injury or mucosal damage, which has remarkable effects of alleviating the pathological development of acute and chronic gastrointestinal diseases, promoting the repair of skin injury or mucosal damage, treating tumors, etc.; and the use of the polypeptide in repairing skin wound or mucosal damage. In particular, the present invention relates to a polypeptide capable of binding with receptor FGFR1, which can inhibit or promote cell proliferation, vascular proliferation, collagen formation, etc., by blocking or activating FGF/FGFR1 signaling.

**Background Art**

At present, it has been found that the family of fibroblast growth factors (FGFs) has at least 19 members, which play an important role in development, angiogenesis and wound healing. In addition, the functions thereof are realized by binding with fibroblast growth factor receptors (FGFRs) and then activating corresponding genes via a signaling system. Fibroblast growth factor receptors are a class of transmembrane tyrosine kinase receptors. Four fibroblast growth factor receptors (FGFR1, FGFR2, FGFR3, and FGFR4) have now been identified, which are encoded by four independent genes. They have similar functions and participate in the regulation of cell proliferation, differentiation and migration and some pathological processes. FGFRs are widely distributed on the surface of target cells, such as epithelial cells, fibroblasts, and vascular endothelial cells. Phenotypic changes of FGFRs are related to hereditary diseases and malignant transformation of cells.

Fibroblast growth factors (FGFs) can activate intracellular signal transduction by binding with their receptors (FGFRs) and regulate cell proliferation, differentiation and migration. Activated FGFRs play an important role in angiogenesis, embryonic development, tumor growth, wound healing, etc.

Skin injury and/or mucosal damage are common pathological characteristics of many diseases. Skin injury refers to damage to normal skin (tissue) caused by external injury-causing factors such as surgery, external forces, heat, electric current, chemicals and low temperatures and internal factors in the body such as local blood supply disturbance. Skin injury is often accompanied by destruction of skin integrity and loss of a certain amount of normal tissues; in addition, the normal function of the skin is impaired. It is also referred to as wound or trauma. At present, proteins/polypeptide drugs, including basic fibroblast growth factor, epidermal growth factor, platelet growth factor, granulocyte-macrophage colony-stimulating factor, growth hormone, etc., have obvious wound healing, skin care, anti-wrinkle, and anti-aging effects. However, these proteins/polypeptide drugs have relatively long amino acid sequences, leading to the disadvantages of high preparation cost and poor stability, so the application thereof is limited to some extent.

Chronic gastritis is a chronic inflammation of gastric mucosa, which is a common and frequently occurring disease in gastroenterology. Clinically, chronic inflammation of gastric mucosa caused by various causes (i.e., manifested as infiltration of monocytes and lymphocytes in pathology) and (or) glandular atrophic lesions are called chronic gastritis. Chronic atrophic gastritis is common in middle-aged and elderly people. The onset thereof is associated with age and has nothing to do with gender. The disease has a slow onset, a lingering tendency, slow recovery, and treatment difficulties.

FGF/FGFR1 signaling is necessary for normal cell growth; however, FGFs may cause many diseases when autosecreted too much or insufficiently expressed. It has been found from research that breast cancer, glioma, liver cancer cells, etc., all have high levels of FGFR1 expression, and FGFR1-mediated signaling abnormalities are closely related to fibrosis diseases such as pulmonary fibrosis and cirrhosis; in addition, when the skin is injured or the mucosa is damaged, activating FGF/FGFR1 signaling can promote cell and tissue damage repair. At present, there are still many difficult problems to be studied and solved by scientists and technicians in the filed of the treatment of skin and mucosal diseases and tumors, and better and more promising drugs are to be found.

In view of the fact that existing treatment means cannot satisfy the needs of clinical treatment, there is still a need for a peptide substance that can better treat skin and mucosal diseases and tumors, such as acute and chronic gastrointestinal diseases and skin injury or mucosal damage diseases.

**Summary of the Invention**

After extensive experiments and research, the inventors have found that the polypeptide of the present invention can bind with receptor FGFR1, and inhibit or promote cell proliferation, vascular proliferation, collagen formation, etc., by blocking or activating FGF/FGFR1 signaling; therefore, the polypeptide of the present invention has remarkable effects of alleviating the pathological development of acute and chronic gastrointestinal diseases, promoting the repair of skin injury or mucosal damage, treating tumors, etc.

In a first aspect, the present invention provides a compound of Formula (I) or a physiologically compatible salt thereof, wherein the compound of Formula (I) is as follows:

$$H\text{-}X_{aa1}\text{-}X_{aa2}\text{-}X_{aa3}\text{-}X_{aa4}\text{-}X_{aa5}\text{-}X_{aa6}\text{-}X_{aa7}\text{-}X_{aa8}\text{-}X_{aa9}\text{-}X_{aa10}\text{-}X_{aa11}\text{-}X_{aa12}\text{-}X_{aa13}\text{-}X_{aa14}\text{-}X_{aa15}\text{-}X_{aa16}\text{-}X_{aa17}\text{-}X_{aa18}\text{-}X_{aa19}\text{-}OH \qquad \textbf{(I)},$$

wherein

$X_{aa1}$ is Pro or absent;
$X_{aa2}$ is Ala or absent;
$X_{aa3}$ is Ser, Ala, Thr or absent;
Xaa4 is Met, Met(O), Val, Ala, Leu, Ile, Thr, Glu, Arg or absent;
$X_{aa5}$ is Gln, Ala, Glu, Pro, Lys or absent;
$X_{aa6}$ is Ala, Ser, Val, Asp or absent;
$X_{aa7}$ is Ser, Ala, Thr, Pro, Val or absent;
$X_{aa8}$ is Leu, Ala, Val, Tyr or absent;
$X_{aa9}$ is Glu, Gln, Ala, Leu, Asp or absent;
$X_{aa10}$ is Ala, Ser or absent;
$X_{aa11}$ is Glu, Gln, Ala, Leu, Asp or absent;
$X_{aa12}$ is Ala, Ser or absent;
$X_{aa13}$ is Lys, Ala, Arg, His or absent;
$X_{aa14}$ is Gly, Ala, Pro or absent;
$X_{aa15}$ is Lys, Arg, Ala, His or absent;
$X_{aa16}$ is Ala, Ser or absent;
$X_{aa17}$ is Glu, Gln, Asp, Asn, Ala, Lys or absent;
$X_{aa18}$ is Asp, Glu, Leu, Ala, Gln or absent; and
$X_{aa19}$ is Asp, Glu or absent; and
provided that at least four of $X_{aa1}$, $X_{aa2}$, $X_{aa3}$, Xaa4, $X_{aa5}$, $X_{aa6}$, $X_{aa7}$, $X_{aa8}$, $X_{aa9}$, $X_{aa10}$, $X_{aa11}$, $X_{aa12}$, $X_{aa13}$, $X_{aa14}$, $X_{aa15}$, $X_{aa16}$, $X_{aa17}$, $X_{aa18}$, and $X_{aa19}$ are not absent.

In one embodiment, $X_{aa1}$ and $X_{aa2}$ are both absent.
In one embodiment, $X_{aa3}$ is Ser or absent, preferably Ser.
In one embodiment, $X_{aa4}$ is Met or Met(O) or absent, preferably Met or Met(O).
In one embodiment, $X_{aa3}$-$X_{aa4}$ is Ser-Met, Ser-Met(O), Ser-Val, Ser-Ala, Ser-Leu, Ser-Ile, Thr -Met or absent, preferably Ser-Met, Ser-Met(O) or absent, more preferably Ser-Met.
In one embodiment, $X_{aa5}$ is Gln or absent, preferably Gln.
In one embodiment, $X_{aa6}$ is Ala or absent, preferably Ala.
In one embodiment, $X_{aa7}$ is Ser or absent, preferably Ser.
In one embodiment, $X_{aa8}$ is Leu or absent, preferably Leu.
In one embodiment, $X_{aa5}$-$X_{aa6}$-$X_{aa7}$-$X_{aa8}$ is Gln-Ala-Ser-Leu, Gln-Ala-Ser-Ala, Ala-Ala-Ser-Leu, Gln-Ala-Ala-Leu, Glu-Ala-Ser-Leu, Gln-Ala-Thr-Leu, Gln-Ala-Ser-Val, Ala-Ser-Thr-Leu, Pro-Val-Pro-Leu, Lys-Asp-Val-Tyr or absent, preferably Gln-Ala-Ser-Leu.
In one embodiment, $X_{aa9}$ is Glu or Gln.
In one embodiment, $X_{aa10}$ is Ala.
In one embodiment, $X_{aa11}$ is Glu, Gln or Ala.
In one embodiment, $X_{aa12}$ is Ala.
In one embodiment, $X_{aa13}$ is Lys.
In one embodiment, $X_{aa9}$-$X_{aa10}$-$X_{aa11}$-$X_{aa12}$ is Glu-Ala-Glu-Ala, Gln-Ala-Gln-Ala, Ala-Ala-Glu-Ala, Glu-Ala-Ala-Ala, Leu-Ala-Glu-Ala, Glu-Ala-Leu-Ala, Gln-Ala-Glu-Ala, Glu-Ala-Gln-Ala, Asp-Ala-Glu-Ala, Glu-Ala-Asp-Ala, Glu-Ser-Glu-Ala, Glu-Ala-Glu-Ser or absent.
In one embodiment, $X_{aa9}$-$X_{aa10}$-$X_{aa11}$-$X_{aa12}$-$X_{aa13}$ is Glu-Ala-Glu-Ala-Lys, Gln-Ala-Gln-Ala-Lys, Glu-Ala-Glu-Ala-Ala, Ala-Ala-Glu-Ala-Lys, Glu-Ala-Ala-Ala-Lys, Glu-Ala-Glu-Ala-Arg, Glu-Ala-Glu-Ala-His, Leu-Ala-Glu-Ala-Lys, Glu-Ala-Leu-Ala-Lys, Gln-Ala-Glu-Ala-Lys, Glu-Ala-Gln-Ala-Lys, Asp-Ala-Glu-Ala-Lys, Glu-Ala-Asp-Ala-Lys, Glu-Ser-Glu-Ala-Lys, Glu-Ala-Glu-Ser-Lys, Glu-Ala-Glu-Ala or absent, preferably Glu-Ala-Glu-Ala-Lys.
In one embodiment, $X_{aa14}$ is Gly.
In one embodiment, $X_{aa15}$ is Lys.
In one embodiment, $X_{aa14}$-$X_{aa15}$ is Gly-Lys, Gly-Arg, Gly-Ala, Gly-His, Ala-Lys, Pro-Lys or absent, preferably Gly-Lys.

In one embodiment, $X_{aa16}$ is Ala.

In one embodiment, $X_{aa17}$ is Glu.

In one embodiment, $X_{aa16}$-$X_{aa17}$ is Ala-Glu, Ala-Gln, Ala-Asp, Ala-Asn, Ala-Ala, Ala-Lys, Ser-Glu or absent.

In one embodiment, $X_{aa18}$-$X_{aa19}$ is Asp, Glu, Leu, Ala, Gln-Asp, Gln-Glu or absent.

In one embodiment, the compound is selected from:

Ser-Met-Gln-Ala-Ser-Leu-Glu-Ala-Glu-Ala-Lys-Gly-Lys-Ala-Glu (Compound 1);

Gln-Ala-Ser-Leu-Glu-Ala-Glu-Ala-Lys-Gly-Lys-Ala-Glu (Compound 2);

Ser-Met-Gln-Ala-Ser-Leu-Glu-Ala-Glu-Ala-Lys-Gly-Lys (Compound 3);

Gln-Ala-Ser-Leu-Glu-Ala-Glu-Ala-Lys-Gly-Lys (Compound 4);

Ser-Met-Gln-Ala-Ser-Leu-Glu-Ala-Glu-Ala-Lys (Compound 5);

Gln-Ala-Ser-Leu-Glu-Ala-Glu-Ala-Lys (Compound 6);

Glu-Ala-Glu-Ala-Lys-Gly-Lys-Ala-Glu (Compound 7);

Ser-Met-Gln-Ala-Ser-Leu (Compound 8);

Gln-Ala-Ser-Leu (Compound 9);

Gly-Lys-Ala-Glu (Compound 10);

Ser-Met(O)-Gln-Ala-Ser-Leu-Glu-Ala-Glu-Ala-Lys-Gly-Lys-Ala-Glu (Compound 11);

Ser-Val-Gln-Ala-Ser-Leu-Glu-Ala-Glu-Ala-Lys-Gly-Lys-Ala-Glu (Compound 12);

Ser-Met-Gln-Ala-Ser-Leu-Glu-Ala-Glu-Ala-Lys-Gly-Lys-Ala-Gln (Compound 13);

Ser-Met-Gln-Ala-Ser-Leu-Gln-Ala-Gln-Ala-Lys-Gly-Lys-Ala-Glu (Compound 14);

Ser-Met-Gln-Ala-Ser-Ala-Glu-Ala-Glu-Ala-Lys-Gly-Lys-Ala-Glu (Compound 15);

Ser-Ala-Gln-Ala-Ser-Leu-Glu-Ala-Glu-Ala-Lys-Gly-Lys-Ala-Glu (Compound 16);

Ser-Leu-Gln-Ala-Ser-Leu-Glu-Ala-Glu-Ala-Lys-Gly-Lys-Ala-Glu (Compound 17);

Ser-Met-Gln-Ala-Ser-Leu-Glu-Ala-Glu-Ala-Ala-Gly-Lys-Ala-Glu (Compound 18);

Ser-Met-Gln-Ala-Ser-Leu-Ala-Ala-Glu-Ala-Lys-Gly-Lys-Ala-Glu (Compound 19);

Ser-Met-Gln-Ala-Ser-Leu-Glu-Ala-Ala-Ala-Lys-Gly-Lys-Ala-Glu (Compound 20);

Ser-Met-Ala-Ala-Ser-Leu-Glu-Ala-Glu-Ala-Lys-Gly-Lys-Ala-Glu (Compound 21);

Ser-Ile-Gln-Ala-Ser-Leu-Glu-Ala-Glu-Ala-Lys-Gly-Lys-Ala-Glu (Compound 22);

Ser-Met-Gln-Ala-Ser-Leu-Glu-Ala-Glu-Ala-Lys-Gly-Lys-Ala-Asp (Compound 23);

Ser-Met-Gln-Ala-Ser-Leu-Glu-Ala-Glu-Ala-Lys-Gly-Lys-Ala-Asn (Compound 24);

Ser-Met-Gln-Ala-Ser-Leu-Glu-Ala-Glu-Ala-Lys-Gly-Arg-Ala-Glu (Compound 25);

Ser-Met-Gln-Ala-Ser-Leu-Glu-Ala-Glu-Ala-Lys-Gly-Ala-Ala-Glu (Compound 26);

Ser-Met-Gln-Ala-Ser-Leu-Glu-Ala-Glu-Ala-Lys-Gly-Lys-Ala-Ala (Compound 27);

Ser-Met-Gln-Ala-Ala-Leu-Glu-Ala-Glu-Ala-Lys-Gly-Lys-Ala-Glu (Compound 28);

Ala-Met-Gln-Ala-Ser-Leu-Glu-Ala-Glu-Ala-Lys-Gly-Lys-Ala-Glu (Compound 29);

Ser-Met-Gln-Ala-Ser-Leu-Glu-Ala-Glu-Ala-Arg-Gly-Lys-Ala-Glu (Compound 30);

Ser-Met-Gln-Ala-Ser-Leu-Glu-Ala-Glu-Ala-Lys-Gly-His-Ala-Glu (Compound 31);

Ser-Met-Gln-Ala-Ser-Leu-Glu-Ala-Glu-Ala-His-Gly-Lys-Ala-Glu (Compound 32);

Ser-Met-Gln-Ala-Ser-Leu-Leu-Ala-Glu-Ala-Lys-Gly-Lys-Ala-Glu (Compound 33);

Ser-Met-Gln-Ala-Ser-Leu-Glu-Ala-Leu-Ala-Lys-Gly-Lys-Ala-Glu (Compound 34);

Ser-Met-Gln-Ala-Ser-Leu-Gln-Ala-Glu-Ala-Lys-Gly-Lys-Ala-Glu (Compound 35);

Ser-Met-Gln-Ala-Ser-Leu-Glu-Ala-Gln-Ala-Lys-Gly-Lys-Ala-Glu (Compound 36);

Ser-Met-Glu-Ala-Ser-Leu-Glu-Ala-Glu-Ala-Lys-Gly-Lys-Ala-Glu (Compound 37);

Ser-Met-Gln-Ala-Ser-Leu-Asp-Ala-Glu-Ala-Lys-Gly-Lys-Ala-Glu (Compound 38);

Ser-Met-Gln-Ala-Ser-Leu-Glu-Ala-Asp-Ala-Lys-Gly-Lys-Ala-Glu (Compound 39);

Thr-Met-Gln-Ala-Ser-Leu-Glu-Ala-Glu-Ala-Lys-Gly-Lys-Ala-Glu (Compound 40);

Ser-Met-Gln-Ala-Thr-Leu-Glu-Ala-Glu-Ala-Lys-Gly-Lys-Ala-Glu (Compound 41);

Ser-Met-Gln-Ala-Ser-Val-Glu-Ala-Glu-Ala-Lys-Gly-Lys-Ala-Glu (Compound 42);

Ser-Met-Gln-Ala-Ser-Leu-Glu-Ser-Glu-Ala-Lys-Gly-Lys-Ala-Glu (Compound 43);

Ser-Met-Gln-Ala-Ser-Leu-Glu-Ala-Glu-Ser-Lys-Gly-Lys-Ala-Glu (Compound 44);

Glu-Ala-Glu-Ala (Compound 45);

Glu-Ala-Glu-Ala-Lys-Gly-Lys (Compound 46);

Thr-Ala-Ser-Thr-Leu-Glu-Ala-Glu-Ala-Lys-Gly-Lys-Ala-Glu (Compound 47);

Ser-Thr-Gln-Ala-Ser-Leu-Glu-Ala-Glu-Ala-Lys-Gly-Lys-Ala-Glu (Compound 48);

Ser-Met-Gln-Ala-Ser-Leu-Glu-Ala-Glu-Ala-Lys-Gly-Lys-Ala-Gln-Asp (Compound 49);

Ser-Met-Gln-Ala-Ser-Leu-Glu-Ala-Glu-Ala-Lys-Gly-Lys-Ala-Gln-Glu (Compound 50);

Ser-Met-Gln-Ala-Ser-Leu-Glu-Ala-Glu-Ala-Lys-Gly-Lys-Ala-Glu-Leu (Compound 51);

Ser-Met-Gln-Ala-Ser-Leu-Glu-Ala-Glu-Ala-Lys-Gly-Lys-Ala-Glu-Ala (Compound 52);
Ser-Met-Gln-Ala-Ser-Leu-Glu-Ala-Glu-Ala-Lys-Gly-Lys-Ala-Lys-Gln-Asp (Compound 53);
Ala-Glu-Pro-Val-Pro-Leu-Glu-Ala-Glu-Ala-Lys-Gly-Lys-Ala-Glu (Compound 54);
Pro-Ala-Ala-Glu-Pro-Val-Pro-Leu-Glu-Ala-Glu-Ala-Lys-Gly-Lys-Ala-Glu (Compound 55);
Ser-Met-Gln-Ala-Ser-Leu-Glu-Ala-Glu-Ala-Lys-Ala-Lys-Ala-Glu (Compound 56);
Ser-Met-Gln-Ala-Ser-Leu-Glu-Ala-Glu-Ala-Lys-Pro-Lys-Ala-Glu (Compound 57);
Ser-Met-Gln-Ala-Ser-Leu-Glu-Ala-Glu-Ala-Lys-Gly-Lys-Ala-Lys-Gln-Glu (Compound 58);
Arg-Lys-Asp-Val-Tyr-Glu-Ala-Glu-Ala-Lys-Gly-Lys-Ala-Glu (Compound 59);
Ser-Met-Gln-Ala-Ser-Leu-Glu-Ala-Glu-Ala-Lys-Gly-Lys-Ser-Glu (Compound 60); or
Glu-Ala-Glu-Ala-Ala-Gly-Lys-Ala-Glu (Compound 61).

In a second aspect, the present invention provides the following compounds or physiologically compatible salts thereof:

Phe-Asp-Ala-Leu-Lys-Gln-Gln-Phe-Gln-Ala-Phe-Gln-Leu-Glu (Compound 62);
His-Cys-Leu-Ala-Gly-Leu-Lys-Lys-Asp-Leu-Glu-Asp-Leu-Glu (Compound 63);
Glu-Ile-Asn-Gln-Leu-Glu-Leu-Ile-Lys-Gln-Ala-Ser-Ile (Compound 64);
Ala-Ala-Arg-Leu-Ala-Asp-Glu-Leu-Arg-Ala-Glu (Compound 65);
Glu-Thr-Leu-Gln-Arg-Lys-Asn-Lys-Glu (Compound 66);
Val-Asp-Ala-Ala-Val-Leu-Glu-Lys-Leu-Glu (Compound 67);
Ala-Ala-Val-Leu-Asp-Lys-Leu-Glu (Compound 68);
Ala-Ala-Val-Leu-Glu-Lys-Leu-Glu (Compound 69);
Lys-Phe-Tyr-Ser-Gln-Ser-Thr-Ala-Ser-Ser-Ser-Tyr-Ala-Tyr-Pro-Ser-His-Phe-Gly-Pro-Ala-Gly-Phe-Ser-Gly-Ser-His-Ser (Compound 70);
Val-Asp-Ala-Ala-Val-Ile-Glu-Lys-Ile-Glu (Compound 71);
Val-Asp-Ala-Ala-Met-Val-Leu-Leu-Thr-Arg (Compound 72); or
Val-Asp-Ala-Ala-Val-Leu-Met-Leu-Arg-Thr (Compound 73).

For convenience, when the compound of the present invention is described in the present application, H on the left side and OH on the right side are omitted.

In a third aspect, the present invention provides a method for repairing skin wound, the method comprising bringing the skin wound into contact with the compound of the present invention (i.e., the compound of the first or second aspect described above) or a physiologically compatible salt thereof; alternatively, the present invention provides the use of the compound of the present invention (i.e., the compound of the first or second aspect described above) or a physiologically compatible salt thereof in the preparation of a medicament or cosmetic for repairing skin wound; alternatively, the present invention provides the compound of the present invention (i.e., the compound of the first or second aspect described above) or a physiologically compatible salt thereof, for repairing skin wound. In one embodiment, the skin wound is related to, but not limited to, epidermal inflammation, mechanical and surgical wound, burns and scalds, ulcers, fistulas, bedsores, and skin injuries caused by radiotherapy and/or chemotherapy. In one embodiment, the skin wound refers to damage to normal skin caused by external injury-causing factors such as surgery, external forces, heat, electric current, chemicals and low temperatures and internal factors in the body such as local blood supply disturbance. In one embodiment, the skin wound is often accompanied by destruction of skin integrity and loss of a certain amount of normal tissues. In another embodiment, the skin wound includes the impairment of the normal function of the skin.

The present invention provides a method for promoting the proliferation of HaCAT cells, the method comprising bringing the cells into contact with the compound of the present invention (i.e., the compound of the first or second aspect described above) or a physiologically compatible salt thereof. Alternatively, the present invention provides the use of the compound of the present invention (i.e., the compound of the first or second aspect described above) or a physiologically compatible salt thereof in the preparation of a medicament for promoting the proliferation of HaCAT cells; alternatively, the present invention provides the compound of the present invention (i.e., the compound of the first or second aspect described above) or a physiologically compatible salt thereof, for promoting the proliferation of HaCAT cells.

In a fourth aspect, the present invention provides a method for repairing mucosal damage, the method comprising administering the compound of the present invention or a physiologically compatible salt thereof to a subject or bringing the mucosal damage into contact with the compound of the present invention or the physiologically compatible salt thereof. Alternatively, the present invention provides the use of the compound of the present invention (i.e., the compound of the first or second aspect described above) or a physiologically compatible salt thereof in the preparation of a medicament for repairing mucosal damage; alternatively, the present invention provides the compound of the present invention (i.e., the compound of the first or second aspect described above) or a physiologically compatible salt thereof, for repairing mucosal damage.

In one embodiment, the mucosal damage is mucosal damage in a cavity such as the digestive system or respiratory system.

The mucosal damage of the digestive system is related to oral, esophageal and gastrointestinal diseases, and oral diseases include oral ulcer, stomatitis, gingivitis, periodontitis, etc.; the esophageal diseases include esophagitis, esophageal ulcer, etc.; and the gastrointestinal diseases include, without limitation, chronic gastritis, chronic atrophic gastritis, acute gastritis, gastroduodenal ulcer, functional gastrointestinal diseases, dyspepsia, precancerous lesions, digestive system tumors, gastrointestinal bleeding, gastroesophageal reflux disease, acute and chronic enteritis, ulcerative colitis, Crohn's disease, and mucosal damages caused by radiotherapy and/or chemotherapy.

In a preferred embodiment, the digestive tract mucosa includes gastric mucosa and intestinal mucosa. In a preferred embodiment, the mucosal damage is gastric mucosal damage caused by an irritant substance or a drug or by a stress state. The irritant substance is, for example, hydrochloric acid, ethanol or alcohol, etc., and the drug is, for example, the non-steroidal anti-inflammatory drug aspirin or indomethacin, etc.

The present invention provides a method for preventing, alleviating or treating a digestive tract disease or eliminating inflammatory edema, the method comprising administering the compound of the present invention or a physiologically compatible salt thereof to a subject. Alternatively, the present invention provides the use of the compound of the present invention (i.e., the compound of the first or second aspect described above) or a physiologically compatible salt thereof in the preparation of a medicament for preventing, alleviating or treating a digestive tract disease or eliminating inflammatory edema; alternatively, the present invention provides the compound of the present invention (i.e., the compound of the first or second aspect described above) or a physiologically compatible salt thereof, for preventing, alleviating or treating a digestive tract disease or eliminating inflammatory edema. The digestive tract disease includes those associated with oral, esophageal and gastrointestinal diseases, and the oral diseases include oral ulcer, stomatitis, gingivitis, periodontitis, etc.; the esophageal diseases include esophagitis, esophageal ulcer, etc.; and the gastrointestinal diseases include, without limitation, chronic gastritis, chronic atrophic gastritis, acute gastritis, gastroduodenal ulcer, functional gastrointestinal diseases, dyspepsia, precancerous lesions, digestive system tumors, gastrointestinal bleeding, gastroesophageal reflux disease, acute and chronic enteritis, ulcerative colitis, Crohn's disease, and mucosal damages caused by radiotherapy and/or chemotherapy. In one embodiment, the prevention, alleviation or treatment of digestive tract disease is carried out by regulating the proliferation and differentiation of stem cells. The method can prevent, alleviate or treat a gastrointestinal disease by means of the protective effect of the compound of the present invention or a physiologically compatible salt thereof on digestive tract mucosae such as gastric mucosa or intestinal mucosa or by repairing the injury of digestive tract mucosae such as gastric mucosa or intestinal mucosa.

The present invention provides a method for repairing skin wound or mucosal damage, the method comprising administering the compound of the present invention (i.e., the compound of the first or second aspect described above) or a physiologically compatible salt thereof to a subject. alternatively, the present invention provides the use of the compound of the present invention (i.e., the compound of the first or second aspect described above) or a physiologically compatible salt thereof in the preparation of a medicament or cosmetic for repairing skin wound or mucosal damage; alternatively, the present invention provides the compound of the present invention (i.e., the compound of the first or second aspect described above) or a physiologically compatible salt thereof, for repairing skin wound or mucosal damage.

The present invention provides a method for blocking or activating FGF/FGFR1 signaling, the method comprising administering the compound of the present invention (i.e., the compound of the first or second aspect described above) or a physiologically compatible salt thereof to a subject, whereby the compound or physiologically compatible salt thereof binds to receptor FGFR1, thus blocking or activating FGF/FGFR1 signaling and inhibiting or promoting cell proliferation, vascular proliferation and collagen formation. Alternatively, the present invention provides the use of the compound of the present invention (i.e., the compound of the first or second aspect described above) or a physiologically compatible salt thereof in the preparation of a medicament for blocking or activating FGF/FGFR1 signaling; alternatively, the present invention provides the compound of the present invention (i.e., the compound of the first or second aspect described above) or a physiologically compatible salt thereof, for blocking or activating FGF/FGFR1 signaling. In a further embodiment, the compound of the present invention (i.e., the compound of the first or second aspect described above) binds to receptor FGFR1, thus blocking or activating FGF/FGFR1 signaling and inhibiting or promoting cell proliferation, vascular proliferation and collagen formation. In one embodiment, the present invention provides a method for activating FGF/FGFR1 signaling, the method comprising administering the compound of the present invention (i.e., the compound of the first or second aspect described above) or a physiologically compatible salt thereof to a subject, whereby the compound or physiologically compatible salt thereof binds to receptor FGFR1, thus activating FGF/FGFR1 signaling and inhibiting or promoting cell proliferation, vascular proliferation and collagen formation; alternatively, the present invention provides the compound of the present invention (i.e., the compound of the first or second aspect described above) or a physiologically compatible salt thereof, for activating FGF/FGFR1 signaling; alternatively, the present invention provides the use of the compound of the present invention (i.e., the compound of the first or second aspect described above) or a physiologically compatible salt thereof in the preparation of a medicament for activating FGF/FGFR1 signaling.

The present invention provides a method for treating a disease associated with FGFR target, said method comprising administering the compound of the present invention (i.e., the compound of the first or second aspect described above) or a physiologically compatible salt thereof to a subject. Alternatively, the present invention provides the use of the compound of the present invention (i.e., the compound of the first or second aspect described above) or a physiologically compatible salt thereof in the preparation of a medicament for treating a disease associated with FGFR target; alternatively, the present invention provides the compound of the present invention (i.e., the compound of the first or second aspect described above) or a physiologically compatible salt thereof, for treating a disease associated with FGFR target. In one embodiment, the FGFR target is FGFR1. In one embodiment, the disease associated with FGFR target is a cancer or tumor.

In the above method of the present invention, the compound of the present invention or a physiologically compatible salt thereof is administered orally, by injection, subcutaneously, etc.

In a fifth aspect, the present invention provides a pharmaceutical composition, food composition, health care or cosmetic composition, or commodity composition, said composition comprising the compound of the present invention or a physiologically compatible salt thereof and a physiologically acceptable carrier. In one embodiment, the physiologically acceptable carrier includes a pharmaceutically acceptable carrier or a cosmetically acceptable carrier. The pharmaceutical composition, food composition, health care or cosmetic composition, or commodity composition can be prepared according to a conventional technique of pharmaceutics or cosmetics, including mixing the compound of the present invention, which acts as an active ingredient, with a carrier, and preparing the mixture into the desired dosage form according to a conventional technique. The composition of the present invention can be formulated into an oral preparation, a mucosal preparation, an injection preparation, an inhalation preparation and a topical preparation as needed.

The polypeptide of the present invention has no homology with known polypeptides, which facilitates artificial polypeptide synthesis to obtain a high-purity polypeptide. Moreover, the polypeptide of the present invention can bind with receptor FGFR1 to exert biological effects and has remarkable therapeutic effects on skin and mucosal diseases of various causes. The polypeptide can be used for treating related diseases such as acute and chronic gastrointestinal diseases and tumors. The polypeptide of the present invention has a relatively small molecular weight, and after oral administration, it has remarkable effects such as eliminating inflammatory edema, promoting the repair of digestive tract mucosal damage, alleviating the pathological development of such gastrointestinal diseases as acute and chronic gastritis and digestive tract ulcers, promoting the repair of skin wound, shortening the wound healing time, modulating immune functions, etc. Oral administration can also work. In addition, the polypeptide of the present invention, when applied to a skin wound surface of the body surface, can function even after disinfection with an iodine preparation or hydrogen peroxide, whereas epidermal growth factors, when applied to the skin of the body surface, will be structurally destroyed and cannot function after disinfection with an iodine preparation or hydrogen peroxide.

**Brief Description of the Drawings**

The drawings of the present application are only for the purpose of further explaining the present application, rather than limiting the scope of the present application.

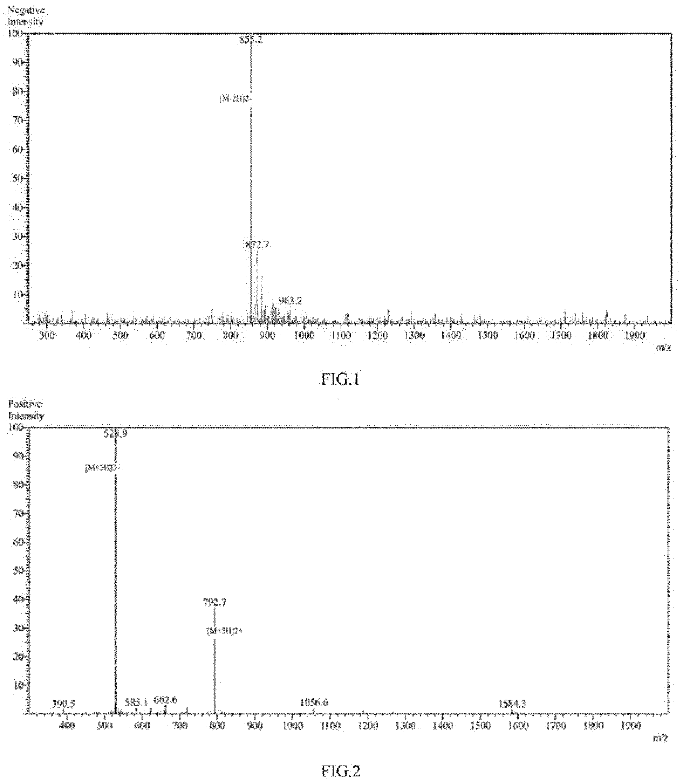

Fig. 1 shows the mass spectrogram of Compound 62.
Fig. 2 shows the mass spectrogram of Compound 63.

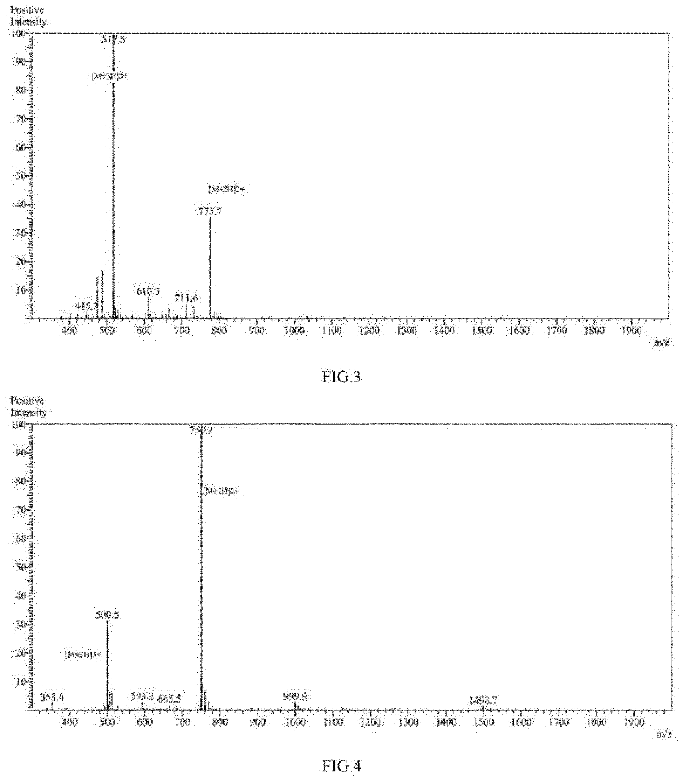

Fig. 3 shows the mass spectrogram of Compound 1.
Fig. 4 shows the mass spectrogram of Compound 64.

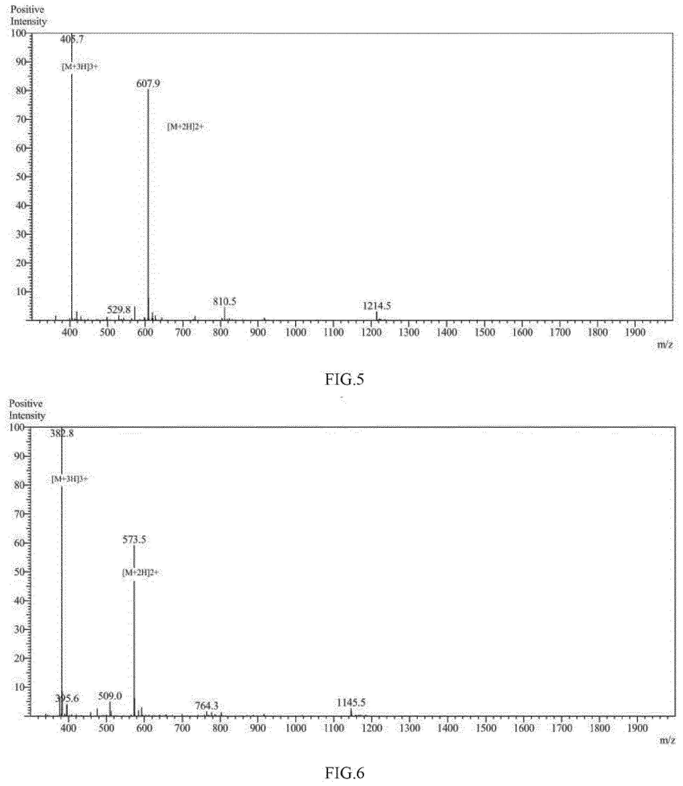

Fig. 5 shows the mass spectrogram of Compound 65.
Fig. 6 shows the mass spectrogram of Compound 66.

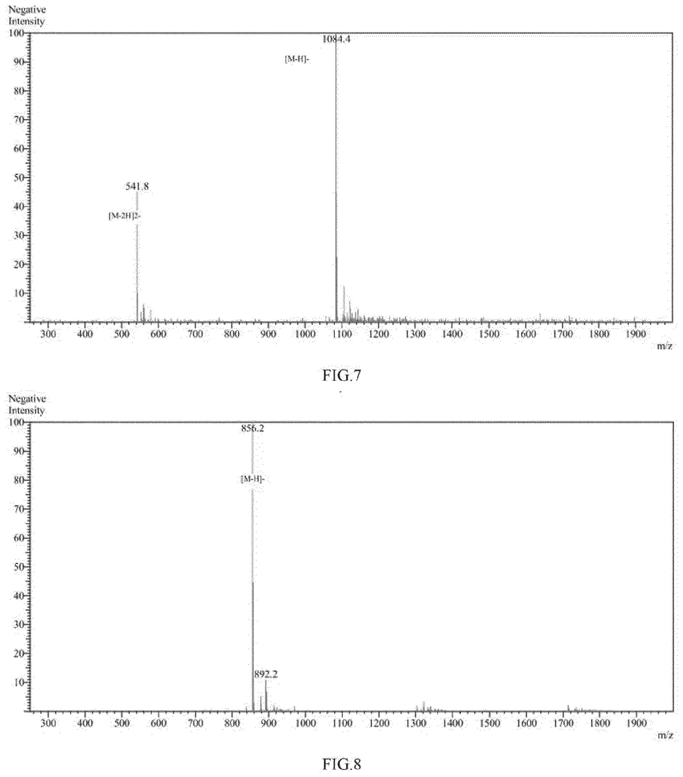

Fig. 7 shows the mass spectrogram of Compound 67.
Fig. 8 shows the mass spectrogram of Compound 68.

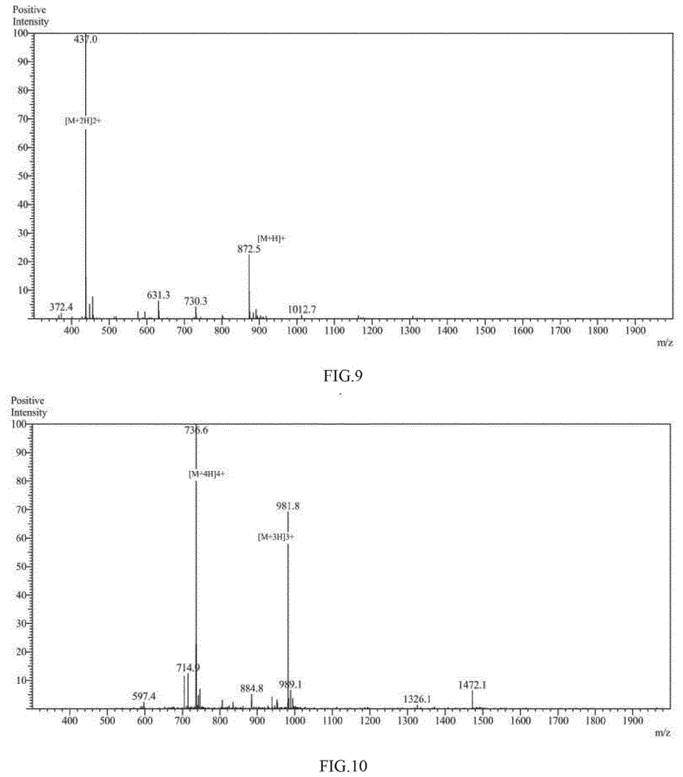

Fig. 9 shows the mass spectrogram of Compound 69.
Fig. 10 shows the mass spectrogram of Compound 70.

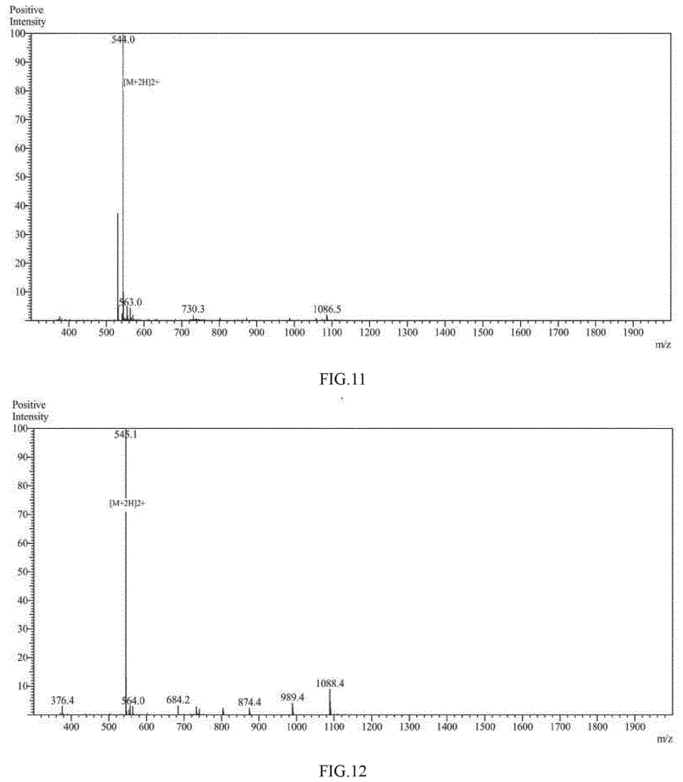

Fig. 11 shows the mass spectrogram of Compound 71.
Fig. 12 shows the mass spectrogram of Compound 72.

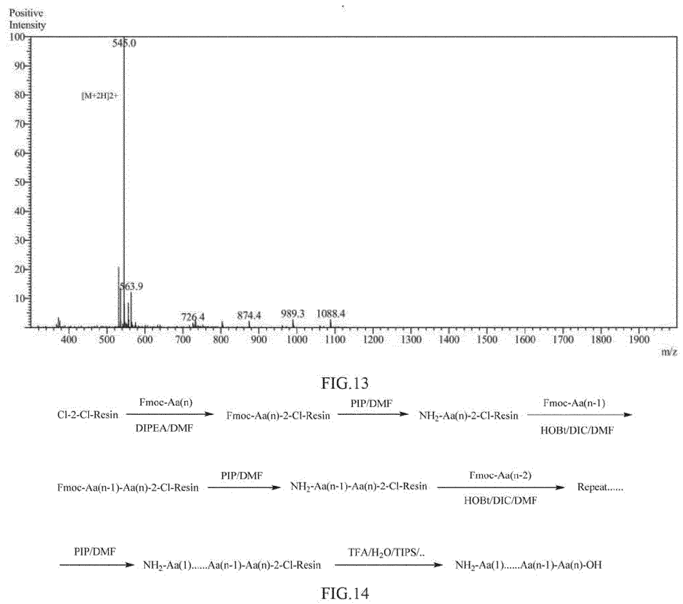

Fig. 13 shows the mass spectrogram of Compound 73.
Fig. 14 shows a schematic diagram of the steps of the solid-phase synthesis of a polypeptide.
Fig. 15 shows the therapeutic effect of Compound 1 on a chronic atrophic gastritis model in mice.
Fig. 16 shows the proliferation-promoting effect of Compound 53 on HaCaT cells.
Fig. 17 shows the proliferation-promoting effect of Compound 1 on HaCaT cells.
Fig. 18 shows the proliferation-promoting effect of Compound 64 on HaCaT cells.

Fig. 19 shows the proliferation-promoting effect of Compound 65 on HaCaT cells.
Fig. 20 shows the proliferation-promoting effect of Compound 66 on HaCaT cells.
Fig. 21 shows the proliferation-promoting effect of Compound 57 on HaCaT cells.
Fig. 22 shows the proliferation-promoting effect of Compound 68 on HaCaT cells.
Fig. 23 shows the proliferation-promoting effect of Compound 69 on HaCaT cells.
Fig. 24 shows the proliferation-promoting effect of Compound 70 on HaCaT cells.
Fig. 25 shows the proliferation-promoting effect of Compound 71 on HaCaT cells.
Fig. 26 shows the proliferation-promoting effect of Compound 72 on HaCaT cells.
Fig. 27 shows the proliferation-promoting effect of Compound 73 on HaCaT cells.
Fig. 28 shows the proliferation-promoting effect of Compound 1 on HMEC-1 cells.
Fig. 29 shows the proliferation-promoting effect of Compound 64 on HMEC-1 cells.
Fig. 30 shows the proliferation-promoting effect of Compound 71 on HMEC-1 cells.
Fig. 31 shows the proliferation-promoting effect of Compound 72 on HMEC-1 cells.
Fig. 32 shows the proliferation-promoting effect of Compound 73 on HMEC-1 cells.
Fig. 33 shows the proliferation-promoting effect of Compound 63 on RSC96 cells.
Fig. 34 shows the proliferation-promoting effect of Compound 1 on RSC96 cells.
Fig. 35 shows the proliferation-promoting effect of Compound 65 on RSC96 cells.
Fig. 36 shows the proliferation-promoting effect of Compound 67 on RSC96 cells.
Fig. 37 shows the effect of Compound 68 on the proliferation of RSC96 cells.
Fig. 38 shows that Compound 70 has an inhibitory effect on the proliferation of RSC96 cells.
Fig. 39 show that effect of Compound 71 on the proliferation of RSC96 cells.
Fig. 40 shows the proliferation-promoting effect of Polypeptide Compound 73 on RSC96 cells.

## Detailed Description of Embodiments

The term "physiologically compatible salt" refers to a salt form that is physiologically compatible (i.e., pharmacologically acceptable) and substantially non-toxic to an individual to whom the compound of the present invention is to be administered. Physiologically compatible salts of the compound of the present invention include conventional and stoichiometric acid addition salts or base addition salts formed from suitable, non-toxic organic or inorganic acids or inorganic bases.

The term "subject" refers to an animal, preferably a mammal, most preferably a human. Specifically, the term "subject" relates to a mammal or human with skin wounds and/or mucosal damage. It should be understood by those skilled in the art that the repair of skin wounds and/or mucosal damage in the present invention can be applied for cosmetic purposes (i.e., non-therapeutic purposes) and therapeutic purposes. To this end, the term "skin injury" in the present application further includes skin injuries to be repaired for cosmetic purposes, such as wrinkles (e.g., wrinkles caused by ultraviolet radiation), skin lines, cracks, lumps, large pores (e.g., those related to accessory structures such as sweat ducts, sebaceous glands or hair follicles), or unevenness or roughness, loss of skin elasticity (loss and/or inactivation of functional skin elastin), sagging (including dropsy of eyes and jaw), loss of skin hardness, loss of skin firmness, loss of recovery ability after skin deformation, discoloration (including dark circles under eyes), macula and blisters, sallow complexion, hyperpigmented skin areas such as senile plaques and freckles, cutin, abnormal differentiation, excessive keratinization, degenerated elastic tissues, destructed collagen, and other tissue changes in skin keratin, dermis, epidermis, skin vascular systems (such as telangiectasia or multi-branched blood vessels) and subcutaneous tissues, especially those close to the skin.

**The following is a description of the present invention in conjunction with specific trials and is not a limitation on the scope of protection of the present invention.**

## Example 1: Chemical Synthesis of Polypeptide

A polypeptide compound was synthesized by a conventional solid-phase synthesis method via multiple cyclic processes of resin swelling, amino acid substitution, deprotection, washing, amino acid activization, condensation, washing, and deprotection, and finally cleavage and side chain deprotection. In the present application, a polypeptide synthesizer was used for synthesis.

Table 1. English names or abbreviations and corresponding Chinese names of solvents, reagents, etc.

| English name or abbreviation | Chinese name |
|---|---|
| 2-Chlorotrityl chloride resin | 2-Chlorotrityl chloride resin |

(continued)

| English name or abbreviation | Chinese name |
|---|---|
| DMF | *N,N*-dimethylformamide |
| DCM | Dichloromethane |
| PIP | Piperidine |
| HOBt | 1-Hydroxybenzotriazole |
| DIPEA | *N,N*-diisopropylethylamine |
| Methanol | Methanol |
| *tert*-Butyl methyl ether | *tert*-Butyl methyl ether |
| TFA | Trifluoroacetic acid |
| TIS | Triisopropylsilane |
| DIC | *N,N'*-diisopropylcarbodiimide |
| Ethanol | Ethanol |
| Aa | Amino acid |

Hereinafter, taking Compound 1 (Ser-Met-Gln-Ala-Ser-Leu-Glu-Ala-Glu-Ala-Lys-Gly-Lys-Ala-Glu) as an example, a method for the synthesis and purification of Compound 1 was shown, the method comprising step 1. preparation of fully protected peptide resin; step 2. cutting and deprotection; and step 3. purification (salt exchange) and freeze-drying.

Step 1. Preparation of fully protected peptide resin

(1) Preparation of Fmoc-Glu(OtBu)-resin: 13.48 g of 2-chlorotrityl chloride resin (SD = 0.75 mmol/g) and dichloromethane (DCM) were weighed and added to six synthesis tubes of a polypeptide synthesizer in order, and a substitution reaction program set on the polypeptide synthesizer was used to carry out a substitution reaction of a first amino acid and remove the protecting group Fmoc. The specific reaction process was as follows: the resin was swollen with DCM for 20 min and then drained. Fmoc-Aa(n)-OH (3 eq, 6 mmol) was dissolved in DMF (40 mL) in an activation flask, and DIPEA (7 eq, 14 mmol) was then added. The mixed solution of Fmoc-Aa(n)-OH/DIPEA in DMF was added to the reaction flask containing the resin, reacted for 1 h and drained. 20% MeOH/DMF (40 mL) was added and reacted for 30 min for endcapping; and after draining, it was washed with DMF 3 times, the protecting group Fmoc was then removed by using 20% PIP/DMF (v/v), and it was then washed with DMF solution 5 times.

(2) Preparation of fully protected peptide resin: The temperature of a circulating water bath was adjusted to 35 degrees Celsius, and amino acids were coupled one by one according to a polypeptide sequence from the (n-1)th amino acid by using a coupling program set on the polypeptide synthesizer. The specific reaction process was as follows: dissolving Fmoc-Aa(n-1)-OH (3 eq, 6 mmol) and HOBt (3 eq, 6 mmol) in DMF (40 mL) in an activation flask, and DIC (4 eq, 8 mmol) was then added for amino acid activation. The mixed solution of Fmoc-Aa(n-1)-OH/HOBt/DIC in DMF was added to the amino acid resin of the previous step for coupling in the reaction flask. After 1 h of reaction, the solvent was discharged, the solid resin was washed with an DMF solution 3 times, the protecting group Fmoc was then removed by using 20% PIP/DMF (v/v), and it was then washed with the DMF solution 5 times. The above steps were repeated until the coupling of the whole amino acid sequence was completed. The amount of each amino acid and condensing agent was shown in Table 2.

Step 2. Cleavage

The resin in the polypeptide synthesizer was transferred to a manual synthesis tube with a sieve plate and washed with DCM 5 times. 200 mL of a cleaving agent (TFA : TIS : $H_2O$ = 95 : 2.5 : 2.5, v/v) was added to the synthesis tube, and it was then bubbled with nitrogen ($N_2$) and reacted for 1.5-3 h.

After the cleavage reaction was complete, the cleaving agent was suction-filtered into a 500 mL roundbottom flask. After concentration in vacuo to a quarter of the volume of the original cleaving agent, 10 folds of the existing volume of methyl *tert*-butyl ether was added, and after settling, a white solid was obtained. The resulting mixture was filtered and washed 3 times separately with 50 mL of methyl *tert*-butyl ether, and the resulting crude peptide product was placed in a vacuum drying oven to remove excess solvent until the crude peptide became a powder. The obtained crude peptide was 11.46 g and had a crude yield of 61.5%.

Step 3. Purification (salt exchange) and freeze-drying

(1) Purification of polypeptide (HPLC)

A. Chromatographic parameters

Chromatographic column: dynamic axial compression column 80*250 mm, packing: Daisogel C18 (SP-100-8-ODS-P).
Eluent A: 0.1% trifluoroacetic acid aqueous solution (v/v)
Eluent B: acetonitrile
Flow rate: 180 mL/min
Ultraviolet detection wavelength: 220 nm

B. Operating steps
a) The crude peptide was dissolved with water and/or acetonitrile, and filtered by a 0.45 $\mu$m filter membrane;
b) sample injection was performed; c) gradient elution was performed with an acetonitrile-water mobile phase;
d) a peptide eluent of interest was collected; and e) rotary evaporation concentration was performed.

(2) Polypeptide salt exchange (HPLC)

A. Chromatographic parameters

Chromatographic column: dynamic axial compression column 80*250 mm, packing: Daisogel C18 (SP-100-8-ODS-P).
Eluent A1: 0.1 M acetic acid
Eluent A2: 0.025 M acetic acid - 0.1 M ammonium acetate
Eluent B: acetonitrile
Flow rate: 180 mL/min
Ultraviolet detection wavelength: 220 nm

B. Operating steps
a) 95% A1 + 5% B balanced chromatographic column; b) sample injection; c) 95% A2 + 5% B balanced chromatographic column; d) gradient elution with A1 and B; e) a peptide eluent of interest was collected; f) rotary evaporation concentration; and g) freeze-drying.

**Table 2. Amounts of amino acids and condensing agents used during chemical synthesis of polypeptides**

| Amino acid name | Aa/mmol | Amino acid amount/g | HOBt/g | DIPEA/g | DIC/g |
|---|---|---|---|---|---|
| Fmoc-L-Glu (OtBu)-OH·H$_2$O | 30 | 13.305 | / | 7.755 | / |
| Fmoc-L-Ala-OH | 30 | 9.882 | 4.7292 | / | 5.048 |
| Fmoc-L-Lys(Boc)-OH | 30 | 14.058 | 4.7292 | / | 5.048 |
| Fmoc-Gly-OH | 30 | 8.919 | 4.7292 | / | 5.048 |
| Fmoc-L-Lys(Boc)-OH | 30 | 14.058 | 4.7292 | / | 5.048 |
| Fmoc-L-Ala-OH | 30 | 9.882 | 4.7292 | / | 5.048 |
| Fmoc-L-Glu (OtBu)-OH·H$_2$O | 30 | 13.305 | 4.7292 | / | 5.048 |
| Fmoc-L-Ala-OH | 30 | 9.882 | 4.7292 | / | 5.048 |
| Fmoc-L-Glu (OtBu)-OH·H$_2$O | 30 | 13.305 | 4.7292 | / | 5.048 |

(continued)

| Amino acid name | Aa/mmol | Amino acid amount/g | HOBt/g | DIPEA/g | DIC/g |
|---|---|---|---|---|---|
| Fmoc-L-Leu-OH | 30 | 10.602 | 4.7292 | / | 5.048 |
| Fmoc-L-Ser(tBu)-OH | 30 | 11.502 | 4.7292 | / | 5.048 |
| Fmoc-L-Ala-OH | 30 | 9.882 | 4.7292 | / | 5.048 |
| Fmoc-Gln(Trt)-OH | 30 | 18.321 | 4.7292 | / | 5.048 |
| Fmoc-L-Met-OH | 30 | 11.145 | 4.7292 | / | 5.048 |
| Fmoc-L-Ser(tBu)-OH | 30 | 11.502 | 4.7292 | / | 5.048 |

Other compounds were synthesized in a similar way to the synthesis of Compound 1. The results were shown in Table 3 and the other parts of the description.

Table 3. Synthesized polypeptide compounds

| No. | Compound sequence | TFA salt crude product (g) | Purification yield | Acetate purity | MS |
|---|---|---|---|---|---|
| 1 | Ser-Met-Gln-Ala-Ser-Leu-Glu-Ala-Glu-Ala-Lys-Gly-Lys-Ala-Glu | 11.46 | NA | 97.43 % | 517.50 (triple charge), 775.60 (double charge) |
| 2 | Gln-Ala-Ser-Leu-Glu-Ala-Glu-Ala-Lys-Gly-Lys-Ala-Glu | 2.37 | 14.3% | 97.45 % | 444.70 (triple charge), 666.50 (double charge), 1331.70 [M+H]$^+$ |
| 3 | Ser-Met-Gln-Ala-Ser-Leu-Glu-Ala-Glu-Ala-Lys-Gly-Lys | 2.66 | NA | 96.81 % | 450.80 (triple charge), 675.60 (double charge), 1349.80 [M+H]$^+$ |
| 4 | Gln-Ala-Ser-Leu-Glu-Ala-Glu-Ala-Lys-Gly-Lys | 2.42 | 24.6% | 95.31 % | 566.50 (double charge), 1131.60 [M+H]$^+$ |
| 5 | Ser-Met-Gln-Ala-Ser-Leu-Glu-Ala-Glu-Ala-Lys | 1.78 | 14.6% | 97.77 % | 583.00 (double charge), 1164.70 [M+H]$^+$ |
| 6 | Gln-Ala-Ser-Leu-Glu-Ala-Glu-Ala-Lys | 1.98 | 21.4% | 98.46 % | 473.90 (double charge), 946.50 [M+H]$^+$ |
| 7 | Glu-Ala-Glu-Ala-Lys-Gly-Lys-Ala-Glu | 1.49 | 6.2% | 96.76 % | 466.90 (double charge), 932.40 [M+H]$^+$ |
| 8 | Ser-Met-Gln-Ala-Ser-Leu | 1.16 | 62.9% | 100% | 636.30 [M+H]$^+$ |
| 9 | Gln-Ala-Ser-Leu | 1.51 | 58.4% | 98.41 % | 418.20 [M+H]$^+$ |
| 10 | Gly-Lys-Ala-Glu | 1.68 | NA | 97.70 % | 404.20 [M+H]$^+$ |
| 11 | Ser-Met(O)-Gln-Ala-Ser-Leu-Glu-Ala-Glu-Ala-Lys-Gly-Lys-Ala-Glu | 2.86 | 28.0% | 97.42 % | 522.90 (triple charge), 783.70 (double charge) |
| 12 | Ser-Val-Gln-Ala-Ser-Leu-Glu-Ala-Glu-Ala-Lys-Gly-Lys-Ala-Glu | 2.42 | 24.3% | 98.27 % | 506.80 (triple charge), 759.70 (double charge) |
| 13 | Ser-Met-Gln-Ala-Ser-Leu-Glu-Ala-Glu-Ala-Lys-Gly-Lys-Ala-Gln | 2.47 | 8.5% | 98.90 % | 517.10 (triple charge), 775.10 (double charge) |

(continued)

| No. | Compound sequence | TFA salt crude product (g) | Purification yield | Acetate purity | MS |
|---|---|---|---|---|---|
| 14 | Ser-Met-Gln-Ala-Ser-Leu-Gln-Ala-Gln-Ala-Lys-Gly-Lys-Ala-Glu | 2.80 | 33.0% | 99.36 % | 516.80 (triple charge), 774.60 (double charge) |
| 15 | Ser-Met-Gln-Ala-Ser-Ala-Glu-Ala-Glu-Ala-Lys-Gly-Lys-Ala-Glu | 2.64 | 7.3% | 99.06 % | 503.40 (triple charge), 754.60 (double charge) |
| 16 | Ser-Ala-Gln-Ala-Ser-Leu-Glu-Ala-Glu-Ala-Lys-Gly-Lys-Ala-Glu | 2.13 | NA | 100% | 497.40 (triple charge), 745.60 (double charge) |
| 17 | Ser-Leu-Gln-Ala-Ser-Leu-Glu-Ala-Glu-Ala-Lys-Gly-Lys-Ala-Glu | 1.57 | NA | 100% | 511.50 (triple charge), 766.60 (double charge) |
| 18 | Ser-Met-Gln-Ala-Ser-Leu-Glu-Ala-Glu-Ala-Ala-Gly-Lys-Ala-Glu | 1.45 | 17.5% | 100% | 498.40 (triple charge), 747.00 (double charge) |
| 19 | Ser-Met-Gln-Ala-Ser-Leu-Ala-Ala-Glu-Ala-Lys-Gly-Lys-Ala-Glu | 1.65 | 12.0% | 96.17 % | 498.10 (triple charge), 746.60 (double charge) |
| 20 | Ser-Met-Gln-Ala-Ser-Leu-Glu-Ala-Ala-Ala-Lys-Gly-Lys-Ala-Glu | 1.53 | 26.1% | 99.01 % | 498.10 (triple charge), 746.50 (double charge) |
| 21 | Ser-Met-Ala-Ala-Ser-Leu-Glu-Ala-Glu-Ala-Lys-Gly-Lys-Ala-Glu | 1.55 | NA | 100% | 498.40 (triple charge), 747.00 (double charge) |
| 22 | Ser-Ile-Gln-Ala-Ser-Leu-Glu-Ala-Glu-Ala-Lys-Gly-Lys-Ala-Glu | 1.38 | 17.2% | 100% | 511.40 (triple charge), 766.50 (double charge) |
| 23 | Ser-Met-Gln-Ala-Ser-Leu-Glu-Ala-Glu-Ala-Lys-Gly-Lys-Ala-Asp | 2.29 | 17.9% | 99.34 % | 512.70 (triple charge), 768.60 (double charge) |
| 24 | Ser-Met-Gln-Ala-Ser-Leu-Glu-Ala-Glu-Ala-Lys-Gly-Lys-Ala-Asn | 2.19 | 4.4% | 98.37 % | 512.40 (triple charge), 768.00 (double charge) |
| 25 | Ser-Met-Gln-Ala-Ser-Leu-Glu-Ala-Glu-Ala-Lys-Gly-Arg-Ala-Glu | 0.45 | 18.2% | 97.28 % | 526.80 (triple charge), 789.60 (double charge) |
| 26 | Ser-Met-Gln-Ala-Ser-Leu-Glu-Ala-Glu-Ala-Lys-Gly-Ala-Ala-Glu | 2.54 | 11.8% | 97.63 % | 498.30 (triple charge), 746.90 (double charge) |
| 27 | Ser-Met-Gln-Ala-Ser-Leu-Glu-Ala-Glu-Ala-Lys-Gly-Lys-Ala-Ala | 2.44 | 37.4% | 98.22 % | 498.00 (triple charge), 746.50 (double charge) |
| 28 | Ser-Met-Gln-Ala-Ala-Leu-Glu-Ala-Glu-Ala-Lys-Gly-Lys-Ala-Glu | 0.64 | 9.1% | 96.05 % | 512.20 (triple charge), 767.60 (double charge) |

(continued)

| No. | Compound sequence | TFA salt crude product (g) | Purification yield | Acetate purity | MS |
|---|---|---|---|---|---|
| 29 | Ala-Met-Gln-Ala-Ser-Leu-Glu-Ala-Glu-Ala-Lys-Gly-Lys-Ala-Glu | 2.92 | NA | 96.94 % | 512.10 (triple charge), 767.50 (double charge) |
| 30 | Ser-Met-Gln-Ala-Ser-Leu-Glu-Ala-Glu-Ala-Arg-Gly-Lys-Ala-Glu | 1.77 | 3.9% | 95.92 % | 526.80 (triple charge), 789.60 (double charge) |
| 31 | Ser-Met-Gln-Ala-Ser-Leu-Glu-Ala-Glu-Ala-Lys-Gly-His-Ala-Glu | 1.49 | 4.1% | 99.74 % | 520.50 (triple charge), 780.10 (double charge) |
| 32 | Ser-Met-Gln-Ala-Ser-Leu-Glu-Ala-Glu-Ala-His-Gly-Lys-Ala-Glu | 1.55 | 1.4% | 98.76 % | 520.50 (triple charge), 780.10 (double charge) |
| 33 | Ser-Met-Gln-Ala-Ser-Leu-Leu-Ala-Glu-Ala-Lys-Gly-Lys-Ala-Glu | 1.55 | 24.6% | 95.50 % | 512.10 (triple charge), 767.60 (double charge) |
| 34 | Ser-Met-Gln-Ala-Ser-Leu-Glu-Ala-Leu-Ala-Lys-Gly-Lys-Ala-Glu | 2.24 | 14.1% | 99.69 % | 512.10 (triple charge), 767.60 (double charge) |
| 35 | Ser-Met-Gln-Ala-Ser-Leu-Gln-Ala-Glu-Ala-Lys-Gly-Lys-Ala-Glu | 2.85 | 8.2% | 98.54 % | 517.10 (triple charge), 775.10 (double charge) |
| 36 | Ser-Met-Gln-Ala-Ser-Leu-Glu-Ala-Gln-Ala-Lys-Gly-Lys-Ala-Glu | 2.01 | 16.5% | 98.89 % | 517.10 (triple charge), 775.10 (double charge) |
| 37 | Ser-Met-Glu-Ala-Ser-Leu-Glu-Ala-Glu-Ala-Lys-Gly-Lys-Ala-Glu | 2.03 | 1.8% | 97.81 % | 517.80 (triple charge), 776.10 (double charge) |
| 38 | Ser-Met-Gln-Ala-Ser-Leu-Asp-Ala-Glu-Ala-Lys-Gly-Lys-Ala-Glu | 2.03 | 4.3% | 97.85 % | 512.80 (triple charge), 768.60 (double charge) |
| 39 | Ser-Met-Gln-Ala-Ser-Leu-Glu-Ala-Asp-Ala-Lys-Gly-Lys-Ala-Glu | 2.30 | 21.1% | 99.74 % | 512.80 (triple charge), 768.50 (double charge) |
| 40 | Thr-Met-Gln-Ala-Ser-Leu-Glu-Ala-Glu-Ala-Lys-Gly-Lys-Ala-Glu | 1.38 | 20.3% | 99.15 % | 522.20 (triple charge), 782.60 (double charge) |
| 41 | Ser-Met-Gln-Ala-Thr-Leu-Glu-Ala-Glu-Ala-Lys-Gly-Lys-Ala-Glu | 2.50 | 19.7% | 100% | 522.10 (triple charge), 782.50 triple charge |
| 42 | Ser-Met-Gln-Ala-Ser-Val-Glu-Ala-Glu-Ala-Lys-Gly-Lys-Ala-Glu | 2.40 | 11.7% | 100% | 512.80 (triple charge), 768.50 (double charge) |
| 43 | Ser-Met-Gln-Ala-Ser-Leu-Glu-Ser-Glu-Ala-Lys-Gly-Lys-Ala-Glu | 2.31 | 9.5% | 98.77 % | 522.80 (triple charge), |

(continued)

| No. | Compound sequence | TFA salt crude product (g) | Purification yield | Acetate purity | MS |
|---|---|---|---|---|---|
| | | | | | 783.50 (double charge) |
| 44 | Ser-Met-Gln-Ala-Ser-Leu-Glu-Ala-Glu-Ser-Lys-Gly-Lys-Ala-Glu | 2.22 | 8.2% | 100% | 522.80 (triple charge), 783.60 (double charge) |
| 45 | Glu-Ala-Glu-Ala | 3.20 | 36.6% | 100% | 419.20 [M+H]+ |
| 46 | Glu-Ala-Glu-Ala-Lys-Gly-Lys | 1.53 | 27.6% | 96.97 % | 366.70 (double charge), 732.30 [M+H]+ |
| 47 | Thr-Ala-Ser-Thr-Leu-Glu-Ala-Glu-Ala-Lys-Gly-Lys-Ala-Glu | 2.24 | 18.9% | 99.74 % | 469.40 (triple charge), 703.60 (double charge) |
| 48 | Ser-Thr-Gln-Ala-Ser-Leu-Glu-Ala-Glu-Ala-Lys-Gly-Lys-Ala-Glu | 2.57 | 25.1% | 98.46 % | 507.40 (triple charge), 760.50 (double charge) |
| 49 | Ser-Met-Gln-Ala-Ser-Leu-Glu-Ala-Glu-Ala-Lys-Gly-Lys-Ala-Gln-Asp | 1.29 | 6.7% | 100% | 555.50 (triple charge), 832.50 (double charge) |
| 50 | Ser-Met-Gln-Ala-Ser-Leu-Glu-Ala-Glu-Ala-Lys-Gly-Lys-Ala-Gln-Glu | 1.63 | 5.2% | 98.43 % | 560.10 (triple charge), 839.50 (double charge) |
| 51 | Ser-Met-Gln-Ala-Ser-Leu-Glu-Ala-Glu-Ala-Lys-Gly-Lys-Ala-Glu-Leu | 2.28 | 5.6% | 100% | 555.10 (triple charge), 832.10 (double charge) |
| 52 | Ser-Met-Gln-Ala-Ser-Leu-Glu-Ala-Glu-Ala-Lys-Gly-Lys-Ala-Glu-Ala | 1.95 | 12.0% | 96.80 % | 541.10 (triple charge), 811.10 (double charge) |
| 53 | Ser-Met-Gln-Ala-Ser-Leu-Glu-Ala-Glu-Ala-Lys-Gly-Lys-Ala-Lys-Gln-Asp | 1.93 | 10.1% | 95.76 % | 598.10 (triple charge), 896.50 (double charge) |
| 54 | Ala-Glu-Pro-Val-Pro-Leu-Glu-Ala-Glu-Ala-Lys-Gly-Lys-Ala-Glu | 2.15 | 36.2% | 99.3% | 513.70 (triple charge), 770.00 (double charge) |
| 55 | Pro-Ala-Ala-Glu-Pro-Val-Pro-Leu-Glu-Ala-Glu-Ala-Lys-Gly-Lys-Ala-Glu | 1.98 | 99.4% | 99.4% | 569.80 (triple charge), 854.10 (double charge) |
| 56 | Ser-Met-Gln-Ala-Ser-Leu-Glu-Ala-Glu-Ala-Lys-Ala-Lys-Ala-Glu | 3.00 | 14.0% | 97.54 % | 522.10 (triple charge), 782.50 (double charge) |
| 57 | Ser-Met-Gln-Ala-Ser-Leu-Glu-Ala-Glu-Ala-Lys-Pro-Lys-Ala-Glu | 2.12 | 21.7% | 99.8% | 530.70 (triple charge), 795.50 (double charge) |
| 58 | Ser-Met-Gln-Ala-Ser-Leu-Glu-Ala-Glu-Ala-Lys-Gly-Lys-Ala-Lys-Gln-Glu | 2.52 | 14.2% | 98.0% | 602.80 (triple charge), 903.60 (double charge) |

(continued)

| No. | Compound sequence | TFA salt crude product (g) | Purification yield | Acetate purity | MS |
|---|---|---|---|---|---|
| 59 | Arg-Lys-Asp-Val-Tyr-Glu-Ala-Glu-Ala-Lys-Gly-Lys-Ala-Glu | 2.16 | 39.4% | 99.71 % | 532.10 (triple charge), 797.50 (double charge) |
| 60 | Ser-Met-Gln-Ala-Ser-Leu-Glu-Ala-Glu-Ala-Lys-Gly-Lys-Ser-Glu | 2.63 | 6.9% | 98.08 % | 522.70 (triple charge), 783.50 (double charge) |
| 61 | Glu-Ala-Glu-Ala-Ala-Gly-Lys-Ala-Glu | 2.10 | 41.1% | 98.36 % | 438.30 (double charge), 875.30 [M+H]$^+$ |

Note: The double charge peak indicated that the molecule of interest bound 2 protons, and the triple charge peak indicated that the molecule of interest bound 3 protons; and N/A represented having difficulties in weighing, and no actual weight was considered.

**Compound 1:** Ser-Met-Gln-Ala-Ser-Leu-Glu-Ala-Glu-Ala-Lys-Gly-Lys-Ala-Glu (acetate)

High-resolution mass spectrometry (TOF-HRMS), molecular formula: $C_{63}H_{108}N_{18}O_{25}S$; m/z: 517.25855 ([M+3H]$^{3+}$), 775.38204 ([M+2H]$^{2+}$), 1549.74923 ([M+H]$^+$).

$^1$H NMR (600 MHz, D$_2$O+MeOD) $\delta$ 4.63 - 4.50 (m, 1H), 4.27 (t, $J$= 7.3 Hz, 1H), 4.19 (dd, $J$= 9.3, 5.1 Hz, 1H), 4.14 (t, $J$= 5.5 Hz, 1H), 4.09 (q, $J$= 7.2 Hz, 2H), 4.05 - 3.93 (m, 9H), 3.85 - 3.76 (m, 2H), 3.75 - 3.62 (m, 4H), 2.75 (t, $J$= 7.6 Hz, 4H), 2.39 - 2.21 (m, 8H), 2.15 (t, $J$= 7.5 Hz, 2H), 2.03 - 1.71 (m, 23H, AcOH), 1.69 - 1.20 (m, 16H), 1.20 - 1.13 (m, 12H), 0.68 (d, $J$ = 6.3 Hz, 3H), 0.62 (d, $J$ = 6.3 Hz, 3H).

**Compound 2:** Gln-Ala-Ser-Leu-Glu-Ala-Glu-Ala-Lys-Gly-Lys-Ala-Glu

High-resolution mass spectrometry (TOF-HRMS), molecular formula: $C_{55}H_{94}N_{16}O_{22}$; m/z: 444.56747 ([M+3H]$^{3+}$), 666.34654 ([M+2H]$^{2+}$), 1331.68030 ([M+H]$^+$).

**Compound 3:** Ser-Met-Gln-Ala-Ser-Leu-Glu-Ala-Glu-Ala-Lys-Gly-Lys

High-resolution mass spectrometry (TOF-HRMS), molecular formula: $C_{55}H_{96}N_{16}O_{21}S$; m/z: 450.56546 ([M+3H]$^{3+}$), 675.34210 ([M+2H]$^{2+}$), 1349.67108 ([M+H]$^+$).

**Compound 4:** Gln-Ala-Ser-Leu-Glu-Ala-Glu-Ala-Lys-Gly-Lys

High-resolution mass spectrometry (TOF-HRMS), molecular formula: $C_{47}H_{82}N_{14}O_{18}$; m/z: 377.87399 ([M+3H]$^{3+}$), 566.30565 ([M+2H]$^{2+}$), 1131.59986 ([M+H]$^+$).

**Compound 5:** Ser-Met-Gln-Ala-Ser-Leu-Glu-Ala-Glu-Ala-Lys

High-resolution mass spectrometry (TOF-HRMS), molecular formula: $C_{47}H_{81}N_{13}O_{19}S$; m/z: 582.78356 ([M+2H]$^{2+}$), 1164.55736 ([M+H]$^{2+}$).

**Compound 6:** Gln-Ala-Ser-Leu-Glu-Ala-Glu-Ala-Lys

High-resolution mass spectrometry (TOF-HRMS), molecular formula: $C_{39}H_{67}N_{11}O_{16}$; m/z: 473.74506 ([M+2H]$^{2+}$), 946.48291 ([M+H]$^{2+}$).

**Compound 7:** Glu-Ala-Glu-Ala-Lys-Gly-Lys-Ala-Glu

High-resolution mass spectrometry (TOF-HRMS), molecular formula: $C_{38}H_{65}N_{11}O_{16}$; m/z: 466.73868 ([M+2H]$^{2+}$), 932.46713 ([M+H]$^+$).

**Compound 8:** Ser-Met-Gln-Ala-Ser-Leu

High-resolution mass spectrometry (TOF-HRMS), molecular formula: $C_{25}H_{45}N_7O_{10}S$; m/z: 636.30254 ([M+H]$^+$).

**Compound 9:** Gln-Ala-Ser-Leu(acetate)

High-resolution mass spectrometry (TOF-HRMS), molecular formula: $C_{17}H_{31}N_5O_7$; m/z: 418.22972 ([M+H]$^+$).
$^1$H NMR (600 MHz, D$_2$O) $\delta$ 4.43 - 4.33 (m, 2H), 4.20 - 4.15 (m, 1H), 4.00 (t, $J$= 6.6 Hz, 1H), 3.85 - 3.76 (m, 2H), 2.46 - 2.35 (m, 2H), 2.17 - 2.04 (m, 2H), 2.00 - 1.96 (m, 2H, AcOH), 1.58 - 1.51 (m, 3H), 1.37 (d, $J$= 7.2 Hz, 3H), 0.89 - 0.76 (m, 6H).

**Compound 10:** Gly-Lys-Ala-Glu

High-resolution mass spectrometry (TOF-HRMS), molecular formula: $C_{16}H_{29}N_5O_7$; m/z: 404.21472 ([M+H]$^+$).

**Compound 11:** Ser-Met(O)-Gln-Ala-Ser-Leu-Glu-Ala-Glu-Ala-Lys-Gly-Lys-Ala-Glu (acetate)

High-resolution mass spectrometry (TOF-HRMS), molecular formula: $C_{63}H_{108}N_{18}O_{26}S$; m/z: 522.58959 ([M+3H]$^{3+}$), 783.37985 ([M+2H]$^{2+}$), 1565.74624 ([M+H]$^+$).
$^1$H NMR (600 MHz, D$_2$O) $\delta$ 4.59 - 4.46 (m, 3H), 4.37 (t, $J$= 5.6 Hz, 1H), 4.32 - 4.22 (m, 7H), 4.22 - 4.17 (m, 3H), 4.16 - 4.10 (m, 2H), 3.98 - 3.92 (m, 2H), 3.90 (s, 1H), 3.88 - 3.77 (m, 3H), 2.94 (t, $J$= 7.5 Hz, 5H), 2.90 - 2.81 (m, 1H), 2.66 (s, 3H), 2.41 - 2.19 (m, 9H), 2.17 - 1.67 (m, 23H, AcOH), 1.66 - 1.53 (m, 7H), 1.48 - 1.36 (m, 4H), 1.35 - 1.32 (m, 12H), 0.87 (d, $J$ = 5.6 Hz, 3H), 0.81 (d, $J$ = 5.7 Hz, 3H).

**Compound 12:** Ser-Val-Gln-Ala-Ser-Leu-Glu-Ala-Glu-Ala-Lys-Gly-Lys-Ala-Glu (acetate)

High-resolution mass spectrometry (TOF-HRMS), molecular formula: $C_{63}H_{108}N_{18}O_{25}$; m/z: 506.59990 ([M+3H]$^{3+}$), 759.39412 ([M+2H]$^{2+}$), 1517.77125 ([M+H]$^+$).
$^1$H NMR (600 MHz, D$_2$O) $\delta$ 4.37 (t, $J$ = 5.5 Hz, 1H), 4.33 - 4.23 (m, 7H), 4.22 - 4.11 (m, 6H), 3.98 - 3.79 (m, 6H), 2.95 (t, $J$= 7.5 Hz, 4H), 2.41 - 2.25 (m, 8H), 2.08 - 2.00 (m, 5H), 1.99 - 1.68 (m, 15H, AcOH), 1.67 - 1.54 (m, 7H), 1.50 - 1.37 (m, 4H), 1.37 - 1.33 (m, 12H), 0.92 - 0.86 (m, 9H), 0.82 (d, $J$ = 5.7 Hz, 3H).

**Compound 13:** Ser-Met-Gln-Ala-Ser-Leu-Glu-Ala-Glu-Ala-Lys-Gly-Lys-Ala-Gln (acetate)

High-resolution mass spectrometry (TOF-HRMS), molecular formula: $C_{63}H_{109}N_{19}O_{24}S$; m/z: 516.92919 ([M+3H]$^{3+}$), 774.88810 ([M+2H]$^{2+}$), 1548.75961 ([M+H]$^+$).
$^1$H NMR (600 MHz, D$_2$O) $\delta$ 4.47 (dd, $J$ = 8.3, 6.1 Hz, 1H), 4.39 - 4.34 (m, 1H), 4.31 - 4.16 (m, 10H), 4.16 - 4.08 (m, 2H), 3.97 - 3.94 (m, 2H), 3.94 - 3.79 (m, 4H), 2.94 (t, $J$ = 7.6 Hz, 4H), 2.60 - 2.47 (m, 2H), 2.40 - 2.22 (m, 8H), 2.10 - 1.98 (m, 9H), 1.98 - 1.84 (m, 13H, AcOH), 1.84 - 1.67 (m, 4H), 1.67 - 1.54 (m, 7H), 1.48 - 1.37 (m, 4H), 1.36 - 1.32 (m, 12H), 0.87 (d, $J$ = 5.8 Hz, 3H), 0.82 (d, $J$ = 5.8 Hz, 3H).

**Compound 14:** Ser-Met-Gln-Ala-Ser-Leu-Gln-Ala-Gln-Ala-Lys-Gly-Lys-Ala-Glu (acetate)

High-resolution mass spectrometry (TOF-HRMS), molecular formula: $C_{63}H_{110}N_{20}O_{23}S$; m/z: 516.60130 ([M+3H]$^{3+}$), 774.39599 ([M+2H]$^{2+}$), 1547.77410 ([M+H]$^+$).
$^1$H NMR (600 MHz, D$_2$O) $\delta$ 4.69 - 4.61 (m, 1H), 4.13 (t, $J$ = 7.3 Hz, 1H), 4.09 - 3.93 (m, 4H), 3.92 - 3.78 (m, 10H), 3.74 - 3.65 (m, 2H), 3.64 - 3.51 (m, 4H), 2.98 - 2.96 (m, 1H), 2.95 - 2.92 (m, 2H), 2.65 - 2.59 (m, 4H), 2.28 - 1.97 (m, 10H), 1.89 - 1.57 (m, 21H, AcOH), 1.57 - 1.18 (m, 11H), 1.18 - 1.08 (m, 4H), 1.07 - 1.00 (m, 12H), 0.55 (d, $J$ = 6.4 Hz, 3H), 0.50 (d, $J$= 6.4 Hz, 3H).

**Compound 15:** Ser-Met-Gln-Ala-Ser-Ala-Glu-Ala-Glu-Ala-Lys-Gly-Lys-Ala-Glu

High-resolution mass spectrometry (TOF-HRMS), molecular formula: $C_{60}H_{102}N_{18}O_{25}S$; m/z: 503.24185 ([M+3H]$^{3+}$), 754.35857 ([M+2H]$^{2+}$), 1507.70677 ([M+H]$^+$).

**Compound 16:** Ser-Ala-Gln-Ala-Ser-Leu-Glu-Ala-Glu-Ala-Lys-Gly-Lys-Ala-Glu

High-resolution mass spectrometry (TOF-HRMS), molecular formula: $C_{61}H_{104}N_{18}O_{25}$; m/z: 497.25701 ([M+3H]

$^{3+}$), 745.38093 ([M+2H]$^{2+}$), 1489.74746 ([M+H]$^+$).

**Compound 17:** Ser-Leu-Gln-Ala-Ser-Leu-Glu-Ala-Glu-Ala-Lys-Gly-Lys-Ala-Glu

High-resolution mass spectrometry (TOF-HRMS), molecular formula: $C_{64}H_{110}N_{18}O_{25}$; m/z: 766.40505 ([M+2H]$^{2+}$).

**Compound 18:** Ser-Met-Gln-Ala-Ser-Leu-Glu-Ala-Glu-Ala-Ala-Gly-Lys-Ala-Glu

High-resolution mass spectrometry (TOF-HRMS), molecular formula: $C_{60}H_{101}N_{17}O_{25}S$; m/z: 746.85331 ([M+2H]$^{2+}$).

**Compound 19:** Ser-Met-Gln-Ala-Ser-Leu-Ala-Ala-Glu-Ala-Lys-Gly-Lys-Ala-Glu

High-resolution mass spectrometry (TOF-HRMS), molecular formula: $C_{61}H_{106}N_{18}O_{23}S$; m/z: 497.92179 ([M+3H]$^{3+}$), 746.37833 ([M+2H]$^{2+}$), 1491.74166 ([M+H]$^+$).

**Compound 20:** Ser-Met-Gln-Ala-Ser-Leu-Glu-Ala-Ala-Ala-Lys-Gly-Lys-Ala-Glu

High-resolution mass spectrometry (TOF-HRMS), molecular formula: $C_{61}H_{106}N_{18}O_{23}S$; m/z: 497.92359 ([M+3H]$^{3+}$), 746.38084 ([M+2H]$^{2+}$), 1491.74509 ([M+H]$^+$).

**Compound 21:** Ser-Met-Ala-Ala-Ser-Leu-Glu-Ala-Glu-Ala-Lys-Gly-Lys-Ala-Glu

High-resolution mass spectrometry (TOF-HRMS), molecular formula: $C_{61}H_{105}N_{17}O_{24}S$; m/z: 498.25060 ([M+3H]$^{3+}$), 746.87141 ([M+2H]$^{2+}$).

**Compound 22:** Ser-Ile-Gln-Ala-Ser-Leu-Glu-Ala-Glu-Ala-Lys-Gly-Lys-Ala-Glu

High-resolution mass spectrometry (TOF-HRMS), molecular formula: $C_{64}H_{110}N_{18}O_{25}$; m/z: 511.27255 ([M+3H]$^{3+}$), 766.40318 ([M+2H]$^{2+}$), 1531.78999 ([M+H]$^+$)

**Compound 23:** Ser-Met-Gln-Ala-Ser-Leu-Glu-Ala-Glu-Ala-Lys-Gly-Lys-Ala-Asp

High-resolution mass spectrometry (TOF-HRMS), molecular formula: $C_{62}H_{106}N_{18}O_{25}S$; m/z: 512.58493 ([M+3H]$^{3+}$), 768.37218 ([M+2H]$^{2+}$), 1535.72417 ([M+H]$^+$)..

**Compound 24:** Ser-Met-Gln-Ala-Ser-Leu-Glu-Ala-Glu-Ala-Lys-Gly-Lys-Ala-Asn

High-resolution mass spectrometry (TOF-HRMS), molecular formula: $C_{62}H_{107}N_{19}O_{24}S$; m/z: 512.25851 ([M+3H]$^{3+}$), 767.88243 ([M+2H]$^{2+}$), 1534.34795 ([M+H]$^+$).

**Compound 25:** Ser-Met-Gln-Ala-Ser-Leu-Glu-Ala-Glu-Ala-Lys-Gly-Arg-Ala-Glu

High-resolution mass spectrometry (TOF-HRMS), molecular formula: $C_{63}H_{108}N_{20}O_{25}S$; m/z: 526.59167 ([M+3H]$^{3+}$), 789.38318 ([M+2H]$^{2+}$), 1577.76111 ([M+H]$^+$).

**Compound 26:** Ser-Met-Gln-Ala-Ser-Leu-Glu-Ala-Glu-Ala-Lys-Gly-Ala-Ala-Glu

High-resolution mass spectrometry (TOF-HRMS), molecular formula: $C_{60}H_{101}N_{17}O_{25}S$; m/z: 746.85430 ([M+2H]$^{2+}$), 1492.68936 ([M+H]$^+$)

**Compound 27:** Ser-Met-Gln-Ala-Ser-Leu-Glu-Ala-Glu-Ala-Lys-Gly-Lys-Ala-Ala

High-resolution mass spectrometry (TOF-HRMS), molecular formula: $C_{61}H_{106}N_{18}O_{23}S$; m/z: 497.92090 ([M+3H]$^{3+}$), 746.37592 ([M+2H]$^{2+}$), 1491.74463 ([M+H]$^+$).

**Compound 28:** Ser-Met-Gln-Ala-Ala-Leu-Glu-Ala-Glu-Ala-Lys-Gly-Lys-Ala-Glu

High-resolution mass spectrometry (TOF-HRMS), molecular formula: $C_{63}H_{108}N_{18}O_{24}S$; m/z: 511.92444 ([M+3H]$^{3+}$), 767.38306 ([M+2H]$^{2+}$), 1533.75671 ([M+H]$^+$).

**Compound 29:** Ala-Met-Gln-Ala-Ser-Leu-Glu-Ala-Glu-Ala-Lys-Gly-Lys-Ala-Glu (acetate)

High-resolution mass spectrometry (TOF-HRMS), molecular formula: $C_{63}H_{108}N_{18}O_{24}S$; m/z: 511.92638 ([M+3H]$^{3+}$), 767.38435 ([M+2H]$^{2+}$), 1533.75280 ([M+H]$^+$).

$^1$H NMR (600 MHz, D$_2$O) $\delta$ 4.38 (dd, $J$= 8.4, 6.0 Hz, 1H), 4.32 (t, $J$ = 5.6 Hz, 1H), 4.26 - 4.12 (m, 10H), 4.08 (dd, $J$ = 8.5, 4.9 Hz, 1H), 4.00 (q, $J$ = 7.1 Hz, 1H), 3.90 - 3.84 (m, 1H), 3.84 - 3.73 (m, 3H), 2.89 (t, $J$ = 7.6 Hz, 4H), 2.54 - 2.42 (m, 2H), 2.37 - 2.21 (m, 8H), 2.05 - 1.95 (m, 8H), 1.95 - 1.49 (m, 25H, AcOH), 1.43 (d, $J$ = 7.1 Hz, 3H), 1.41 - 1.31 (m, 4H), 1.30 (t, $J$ = 7.2 Hz, 12H), 0.83 (d, $J$ = 5.8 Hz, 3H), 0.77 (d, $J$ = 6.0 Hz, 3H).

**Compound 30:** Ser-Met-Gln-Ala-Ser-Leu-Glu-Ala-Glu-Ala-Arg-Gly-Lys-Ala-Glu

High-resolution mass spectrometry (TOF-HRMS), molecular formula: $C_{63}H_{108}N_{20}O_{25}S$; 526.59256 ([M+3H]$^{3+}$), 789.38355 ([M+2H]$^{2+}$), 1577.74446 ([M+H]$^+$).

**Compound 31:** Ser-Met-Gln-Ala-Ser-Leu-Glu-Ala-Glu-Ala-Lys-Gly-His-Ala-Glu

High-resolution mass spectrometry (TOF-HRMS), molecular formula: $C_{63}H_{103}N_{19}O_{25}S$; m/z: 520.24450 ([M+3H]$^{3+}$), 779.86149 ([M+2H]$^{2+}$), 1558.70931 ([M+H]$^+$).

**Compound 32:** Ser-Met-Gln-Ala-Ser-Leu-Glu-Ala-Glu-Ala-His-Gly-Lys-Ala-Glu

High-resolution mass spectrometry (TOF-HRMS), molecular formula: $C_{63}H_{103}N_{19}O_{25}S$; m/z: 520.24531 ([M+3H]$^{3+}$), 779.86349 ([M+2H]$^{2+}$), 1558.71094 ([M+H]$^+$).

**Compound 33:** Ser-Met-Gln-Ala-Ser-Leu-Leu-Ala-Glu-Ala-Lys-Gly-Lys-Ala-Glu

High-resolution mass spectrometry (TOF-HRMS), molecular formula: $C_{64}H_{112}N_{18}O_{23}S$; m/z: 767.38324 ([M+2H]$^{2+}$), 1533.75610 ([M+H]$^+$).

**Compound 34:** Ser-Met-Gln-Ala-Ser-Leu-Glu-Ala-Leu-Ala-Lys-Gly-Lys-Ala-Glu (acetate)

High-resolution mass spectrometry (TOF-HRMS), molecular formula: $C_{64}H_{112}N_{18}O_{23}S$; m/z: 767.39618 ([M+2H]$^{2+}$), 1533.79517 ([M+H]$^+$).

$^1$H NMR (600 MHz, D$_2$O) $\delta$ 4.39 (dd, $J$ = 8.2, 6.2 Hz, 1H), 4.30 (dd, $J$ = 9.3, 5.1 Hz, 1H), 4.26 (t, $J$ = 5.4 Hz, 1H), 4.23 - 4.04 (m, 11H), 3.89 (d, $J$ = 4.7 Hz, 2H), 3.87 - 3.74 (m, 4H), 2.86 (t, $J$ = 7.7 Hz, 4H), 2.50 - 2.40 (m, 2H), 2.40 - 2.32 (m, 4H), 2.26 (t, $J$ = 7.5 Hz, 2H), 2.13 - 2.05 (m, 1H), 2.00 - 1.96 (m, 5H), 1.96 - 1.83 (m, 13H, AcOH), 1.78 - 1.60 (m, 4H), 1.60 - 1.51 (m, 8H), 1.51 - 1.43 (m, 2H), 1.42 - 1.29 (m, 4H), 1.29 - 1.26 (m, 12H), 0.80 (t, $J$ = 5.6 Hz, 6H), 0.74 (d, $J$ = 6.0 Hz, 6H).

**Compound 35:** Ser-Met-Gln-Ala-Ser-Leu-Gln-Ala-Glu-Ala-Lys-Gly-Lys-Ala-Glu

High-resolution mass spectrometry (TOF-HRMS), molecular formula: $C_{63}H_{109}N_{19}O_{24}S$; m/z: 774.88964 ([M+2H]$^{2+}$), 1548.76276 ([M+H]$^+$).

**Compound 36:** Ser-Met-Gln-Ala-Ser-Leu-Glu-Ala-Gln-Ala-Lys-Gly-Lys-Ala-Glu

High-resolution mass spectrometry (TOF-HRMS), molecular formula: $C_{63}H_{109}N_{19}O_{24}S$; m/z: 516.92993 ([M+3H]$^{3+}$), 774.88916 ([M+2H]$^{2+}$), 1548.76034 ([M+H]$^+$).

**Compound 37:** Ser-Met-Glu-Ala-Ser-Leu-Glu-Ala-Glu-Ala-Lys-Gly-Lys-Ala-Glu

High-resolution mass spectrometry (TOF-HRMS), molecular formula: $C_{63}H_{107}N_{17}O_{26}S$; m/z: 775.87388 ([M+2H]$^{2+}$), 1550.72354 ([M+H]$^+$).

**Compound 38:** Ser-Met-Gln-Ala-Ser-Leu-Asp-Ala-Glu-Ala-Lys-Gly-Lys-Ala-Glu

High-resolution mass spectrometry (TOF-HRMS), molecular formula: $C_{62}H_{106}N_{18}O_{25}S$; m/z: 768.37273 ($[M+2H]^{2+}$), 1535.72499 ($[M+H]^+$).

**Compound 39:** Ser-Met-Gln-Ala-Ser-Leu-Glu-Ala-Asp-Ala-Lys-Gly-Lys-Ala-Glu

High-resolution mass spectrometry (TOF-HRMS), molecular formula: $C_{62}H_{106}N_{18}O_{25}S$; m/z: 768.37189 ($[M+2H]^{2+}$), 1535.72715 ($[M+H]^+$).

**Compound 40:** Thr-Met-Gln-Ala-Ser-Leu-Glu-Ala-Glu-Ala-Lys-Gly-Lys-Ala-Glu

High-resolution mass spectrometry (TOF-HRMS), molecular formula: $C_{64}H_{110}N_{18}O_{25}S$; m/z: 521.92978 ($[M+3H]^{3+}$), 782.38824 ($[M+2H]^{2+}$), 1563.75498 ($[M+H]^+$).

**Compound 41:** Ser-Met-Gln-Ala-Thr-Leu-Glu-Ala-Glu-Ala-Lys-Gly-Lys-Ala-Glu

High-resolution mass spectrometry (TOF-HRMS), molecular formula: $C_{64}H_{110}N_{18}O_{25}S$; m/z: 521.92871 ($[M+3H]^{3+}$), 782.38721 ($[M+2H]^{2+}$), 1563.76697 ($[M+H]^+$).

**Compound 42:** Ser-Met-Gln-Ala-Ser-Val-Glu-Ala-Glu-Ala-Lys-Gly-Lys-Ala-Glu

High-resolution mass spectrometry (TOF-HRMS), molecular formula: $C_{62}H_{106}N_{18}O_{25}S$; m/z: 768.37357 ($[M+2H]^{2+}$).

**Compound 43:** Ser-Met-Gln-Ala-Ser-Leu-Glu-Ser-Glu-Ala-Lys-Gly-Lys-Ala-Glu

High-resolution mass spectrometry (TOF-HRMS), molecular formula: $C_{63}H_{108}N_{18}O_{26}S$; m/z: 522.58964 ($[M+3H]^{3+}$), 783.37864 ($[M+2H]^{2+}$).

**Compound 44:** Ser-Met-Gln-Ala-Ser-Leu-Glu-Ala-Glu-Ser-Lys-Gly-Lys-Ala-Glu

High-resolution mass spectrometry (TOF-HRMS), molecular formula: $C_{63}H_{108}N_{18}O_{26}S$; m/z: 783.37737 ($[M+2H]^{2+}$), 1565.72717 ($[M+H]^+$).

**Compound 45:** Glu-Ala-Glu-Ala (acetate)

High-resolution mass spectrometry (TOF-HRMS), molecular formula: $C_{16}H_{26}N_4O_9$; m/z: 419.17728 ($[M+H]^+$).
$^1$H NMR (600 MHz, DMSO-d6) $\delta$ 8.60 (d, $J$ = 7.1 Hz, 1H), 8.27 (d, $J$ = 7.8 Hz, 1H), 7.90 (d, $J$ = 6.8 Hz, 1H), 4.29 (p, $J$ = 7.0 Hz, 1H), 4.19 (td, $J$ = 8.3, 5.1 Hz, 1H), 4.02 (p, $J$ = 7.1 Hz, 1H), 3.66 (t, $J$ = 6.3 Hz, 1H), 2.31 (t, $J$ = 7.6 Hz, 2H), 2.23 (t, $J$ = 7.9 Hz, 2H), 1.95 - 1.79 (m, 5H, AcOH), 1.79 - 1.70 (m, 1H), 1.27 - 1.21 (m, 6H).

**Compound 46:** Glu-Ala-Glu-Ala-Lys-Gly-Lys

High-resolution mass spectrometry (TOF-HRMS), molecular formula: $C_{30}H_{53}N_9O_{12}$; m/z: 732.38880 ($[M+H]^+$).

**Compound 47:** Thr-Ala-Ser-Thr-Leu-Glu-Ala-Glu-Ala-Lys-Gly-Lys-Ala-Glu

High-resolution mass spectrometry (TOF-HRMS), molecular formula: $C_{58}H_{100}N_{16}O_{24}$; m/z: 469.24518 ($[M+3H]^{3+}$), 703.36326 ($[M+2H]^{2+}$), 1405.71314 ($[M+H]^+$).

**Compound 48:** Ser-Thr-Gln-Ala-Ser-Leu-Glu-Ala-Glu-Ala-Lys-Gly-Lys-Ala-Glu (acetate)

High-resolution mass spectrometry (TOF-HRMS), molecular formula: $C_{62}H_{106}N_{18}O_{26}$; m/z: 507.25973 ($[M+3H]^{3+}$), 760.38380 ($[M+2H]^{2+}$), 1519.74995 ($[M+H]^+$).
$^1$H NMR (600 MHz, D$_2$O) $\delta$ 4.33 - 4.27 (m, 2H), 4.25 - 4.11 (m, 12H), 4.08 (dd, $J$ = 8.6, 4.9 Hz, 1H), 3.95 - 3.89 (m, 2H), 3.89 - 3.85 (m, 1H), 3.84 - 3.73 (m, 3H), 2.89 (t, $J$ = 7.6 Hz, 4H), 2.39 - 2.22 (m, 8H), 2.04 - 1.86 (m, 20H, AcOH), 1.86 - 1.63 (m, 5H), 1.63 - 1.46 (m, 7H), 1.43 - 1.32 (m, 4H), 1.31 - 1.27 (m, 12H), 1.13 (d, $J$ = 6.3 Hz, 3H), 0.81 (d, $J$ = 6.2 Hz, 3H), 0.76 (d, $J$ = 6.2 Hz, 3H).

**Compound 49:** Ser-Met-Gln-Ala-Ser-Leu-Glu-Ala-Glu-Ala-Lys-Gly-Lys-Ala-Gln-Asp

High-resolution mass spectrometry (TOF-HRMS), molecular formula: $C_{67}H_{114}N_{20}O_{27}S$; m/z: 555.27329 ($[M+3H]^{3+}$), 832.40392 ($[M+2H]^{2+}$), 1663.79211 ($[M+H]^+$).

**Compound 50:** Ser-Met-Gln-Ala-Ser-Leu-Glu-Ala-Glu-Ala-Lys-Gly-Lys-Ala-Gln-Glu

High-resolution mass spectrometry (TOF-HRMS), molecular formula: $C_{68}H_{116}N_{20}O_{27}S$; m/z: 559.94388 ($[M+3H]^{3+}$), 839.40987 ($[M+2H]^{2+}$), 1677.80026 ($[M+H]^+$).

**Compound 51:** Ser-Met-Gln-Ala-Ser-Leu-Glu-Ala-Glu-Ala-Lys-Gly-Lys-Ala-Gln-Leu

High-resolution mass spectrometry (TOF-HRMS), molecular formula: $C_{69}H_{119}N_{19}O_{26}S$; m/z: 554.95315 ($[M+3H]^{3+}$), 831.92378 ($[M+2H]^{2+}$).

**Compound 52:** Ser-Met-Gln-Ala-Ser-Leu-Glu-Ala-Glu-Ala-Lys-Gly-Lys-Ala-Gln-Ala

High-resolution mass spectrometry (TOF-HRMS), molecular formula: $C_{66}H_{113}N_{19}O_{26}S$; m/z: 540.93567 ($[M+3H]^{3+}$), 810.89807 ($[M+2H]^{2+}$).

**Compound 53:** Ser-Met-Gln-Ala-Ser-Leu-Glu-Ala-Glu-Ala-Lys-Gly-Lys-Ala-Lys-Gln-Asp

High-resolution mass spectrometry (TOF-HRMS), molecular formula: $C_{73}H_{126}N_{22}O_{28}S$; m/z: 448.73124 ($[M+4H]^{4+}$), 597.97133 ($[M+3H]^{3+}$), 896.45075 ($[M+2H]^{2+}$), 1791.88031 ($[M+H]^+$).

**Compound 54:** Ala-Glu-Pro-Val-Pro-Leu-Glu-Ala-Glu-Ala-Lys-Gly-Lys-Ala-Glu

High-resolution mass spectrometry (TOF-HRMS), molecular formula: $C_{67}H_{111}N_{17}O_{24}$; m/z: 513.60834 ($[M+3H]^{3+}$), 769.90656 ($[M+2H]^{2+}$), 1538.80615 ($[M+H]^+$).

**Compound 55:** Pro-Ala-Ala-Glu-Pro-Val-Pro-Leu-Glu-Ala-Glu-Ala-Lys-Gly-Lys-Ala-Glu

High-resolution mass spectrometry (TOF-HRMS), molecular formula: $C_{75}H_{123}N_{19}O_{26}$; m/z: 569.63794 ($[M+3H]^{3+}$), 853.95032 ($[M+2H]^{2+}$), 1706.89331 ($[M+H]^+$).

**Compound 56:** Ser-Met-Gln-Ala-Ser-Leu-Glu-Ala-Glu-Ala-Lys-Ala-Lys-Ala-Glu

High-resolution mass spectrometry (TOF-HRMS), molecular formula: $C_{64}H_{110}N_{18}O_{25}S$; m/z: 521.93003 ($[M+3H]^{3+}$), 782.38948 ($[M+2H]^{2+}$), 1563.76203 ($[M+H]^+$).

**Compound 57:** Ser-Met-Gln-Ala-Ser-Leu-Glu-Ala-Glu-Ala-Lys-Pro-Lys-Ala-Glu (acetate)

High-resolution mass spectrometry (TOF-HRMS), molecular formula: $C_{66}H_{112}N_{18}O_{25}S$; m/z: 530.60034 ($[M+3H]^{3+}$), 795.39502 ($[M+2H]^{2+}$), 1589.78271 ($[M+H]^+$).
$^1$H NMR (600 MHz, D$_2$O) $\delta$ 4.49 (dd, $J$ = 9.1, 5.2 Hz, 1H), 4.42 (dd, $J$ = 8.4, 5.9 Hz, 1H), 4.31 (q, $J$ = 7.4, 6.4 Hz, 2H), 4.26 - 4.13 (m, 9H), 4.11 - 4.05 (m, 2H), 3.94 - 3.86 (m, 2H), 3.83 - 3.69 (m, 3H), 3.55 - 3.47 (m, 1H), 2.93 - 2.87 (m, 4H), 2.54 - 2.42 (m, 2H), 2.36 - 2.16 (m, 9H), 2.04 - 1.93 (m, 9H), 1.93 - 1.49 (m, 28H, AcOH), 1.43 - 1.32 (m, 4H), 1.31 - 1.25 (m, 12H), 0.82 (d, $J$ = 5.7 Hz, 3H), 0.76 (d, $J$ = 5.7 Hz, 3H).

**Compound 58:** Ser-Met-Gln-Ala-Ser-Leu-Glu-Ala-Glu-Ala-Lys-Gly-Lys-Ala-Lys-Gln-Glu

High-resolution mass spectrometry (TOF-HRMS), molecular formula: $C_{74}H_{128}N_{22}O_{28}S$; m/z: 452.23230 ($[M+4H]^{4+}$), 602.64038 ($[M+3H]^{3+}$), 903.45508 ($[M+2H]^{2+}$), 1805.90295 ($[M+H]^+$).

**Compound 59:** Arg-Lys-Asp-Val-Tyr-Glu-Ala-Glu-Ala-Lys-Gly-Lys-Ala-Glu

High-resolution mass spectrometry (TOF-HRMS), molecular formula: $C_{68}H_{112}N_{20}O_{24}$; m/z: 399.21298 ([M+4H]$^{4+}$), 531.94670 ([M+3H]$^{3+}$), 797.41529 ([M+2H]$^{2+}$), 1593.81299 ([M+H]$^+$).

**Compound 60:** Ser-Met-Gln-Ala-Ser-Leu-Glu-Ala-Glu-Ala-Lys-Gly-Lys-Ser-Glu (acetate)

High-resolution mass spectrometry (TOF-HRMS), molecular formula: $C_{63}H_{108}N_{18}O_{26}S$; m/z: 522.58909 ([M+3H]$^{3+}$), 783.37789 ([M+2H]$^{2+}$), 1565.73801 ([M+H]$^+$).

$^1$H NMR (600 MHz, D$_2$O) $\delta$ 4.45 - 4.39 (m, 1H), 4.37 (t, $J$ = 5.5 Hz, 1H), 4.34 - 4.26 (m, 2H), 4.25 - 4.06 (m, 10H), 3.91 - 3.90 (m, 2H), 3.88 - 3.86 (m, 1H), 3.85 - 3.80 (m, 2H), 3.79 - 3.74 (m, 3H), 2.89 (t, $J$ = 7.5 Hz, 4H), 2.54 - 2.43 (m, 1H), 2.39 - 2.22 (m, 7H), 2.06 - 1.96 (m, 8H), 1.95 - 1.92 (m, 23H, AcOH), 1.91 - 1.87 (m, 2H), 1.86 - 1.73 (m, 2H), 1.72 - 1.65 (m, 1H), 1.62 - 1.54 (m, 6H), 1.54 - 1.48 (m, 1H), 1.45 - 1.31 (m, 4H), 1.30 (d, $J$ = 7.2 Hz, 9H), 0.82 (d, $J$ = 5.9 Hz, 3H), 0.77 (d, $J$ = 5.9 Hz, 3H).

**Compound 61:** Glu-Ala-Glu-Ala-Ala-Gly-Lys-Ala-Glu (acetate)

High-resolution mass spectrometry (TOF-HRMS), molecular formula: $C_{35}H_{58}N_{10}O_{16}$; m/z: 438.21012 ([M+2H]$^{2+}$), 875.40922 ([M+H]$^+$).

$^1$H NMR (600 MHz, D$_2$O) $\delta$ 4.29 - 4.15 (m, 7H), 4.11 (dd, $J$ = 8.6, 4.9 Hz, 1H), 3.95 (t, $J$ = 6.5 Hz, 1H), 3.87 - 3.77 (m, 2H), 2.88 (t, $J$ = 7.6 Hz, 2H), 2.39 - 2.25 (m, 7H), 2.10 - 1.94 (m, 9H, AcOH), 1.91 - 1.78 (m, 2H), 1.78 - 1.69 (m, 1H), 1.69 - 1.62 (m, 1H), 1.62 - 1.53 (m, 2H), 1.37 - 1.30 (m, 2H), 1.30 - 1.25 (m, 14H).

**Compound 62:** Phe-Asp-Ala-Leu-Lys-Gln-Gln-Phe-Gln-Ala-Phe-Gln-Leu-Glu

High-resolution mass spectrometry (Orbitrap Exploris) m/z: 571.62579 ([M+3H]$^{3+}$), 856.93494 ([M+2H]$^{2+}$)

**Compound 63:** His-Cys-Leu-Ala-Gly-Leu-Lys-Lys-Asp-Leu-Glu-Asp-Leu-Glu

High-resolution mass spectrometry (Orbitrap Exploris) m/z: 528.60779 ([M+3H]$^{3+}$), 792.40802 ([M+2H]$^{2+}$)

**Compound 64:** Glu-Ile-Asn-Gln-Leu-Glu-Leu-Ile-Lys-Gln-Ala-Ser-Ile

High-resolution mass spectrometry (Orbitrap Exploris) m/z 500.62137 ([M+3H]$^{3+}$), 749.92682 ([M+2H]$^{2+}$), 1499.85291 ([M+H]$^+$)

**Compound 65:** Ala-Ala-Arg-Leu-Ala-Asp-Glu-Leu-Arg-Ala-Glu

High-resolution mass spectrometry (Orbitrap Exploris) m/z: 405.55411 ([M+3H]$^{3+}$), 607.82733 ([M+2H]$^{2+}$), 1214.64624 ([M+H]$^+$)

**Compound 66:** Glu-Thr-Leu-Gln-Arg-Lys-Asn-Lys-Glu

High-resolution mass spectrometry (Orbitrap Exploris) m/z: 382.54669 ([M+3H]$^{3+}$), 573.31622 ([M+2H]$^{2+}$), 1146.61548 ([M+H]$^+$)

**Compound 67:** Val-Asp-Ala-Ala-Val-Leu-Glu-Lys-Leu-Glu

High-resolution mass spectrometry (Orbitrap Exploris) m/z: 543.80505 ([M+2H]$^{2+}$), 1086.60242 ([M+H]$^+$)

**Compound 68:** Ala-Ala-Val-Leu-Asp-Lys-Leu-Glu

High-resolution mass spectrometry (Orbitrap Exploris) m/z: 429.74985 ([M+2H]$^{2+}$), 858.49243 ([M+H]$^+$)

**Compound 69:** Ala-Ala-Val-Leu-Glu-Lys-Leu-Glu

High-resolution mass spectrometry (Orbitrap Exploris) m/z: 436.75772 ([M+2H]$^{2+}$), 872.50806 ([M+H]$^+$)

**Compound 70:** Lys-Phe-Tyr-Ser-Gln-Ser-Thr-Ala-Ser-Ser-Ser-Tyr-Ala-Tyr-Pro-Ser-His-Phe-Gly-Pro-Ala-Gly-Phe-Ser-Gly-Ser-His-Ser

High-resolution mass spectrometry (Orbitrap Exploris) m/z: 736.58075 ([M+4H]4+), 981.77148 ([M+3H]$^{3+}$), 1472.15320 ([M+2H]$^{2+}$)

**Compound 71:** Val-Asp-Ala-Ala-Val-Ile-Glu-Lys-Ile-Glu

High-resolution mass spectrometry (Orbitrap Exploris) m/z: 543.80481 ([M+2H]$^{2+}$), 1086.60217 ([M+H]$^+$)

**Compound 72:** Val-Asp-Ala-Ala-Met-Val-Leu-Leu-Thr-Arg

High-resolution mass spectrometry (Orbitrap Exploris) m/z: 544.80920 ([M+2H]$^{2+}$), 1088.61121 ([M+H]$^+$)

**Compound 73:** Val-Asp-Ala-Ala-Val-Leu-Met-Leu-Arg-Thr

High-resolution mass spectrometry (Orbitrap Exploris) m/z: 544.80945 ([M+2H]$^{2+}$), 1088.61169 ([M+H]$^+$)

**Example 2: FGFR receptor binding assay: experiment of affinity to FGFR1 protein target**

The affinity of the polypeptide compound prepared by the present invention to FGFR1 was tested by using a control FGFR1 allosteric inhibitor (product number SSR128129E from Sigma) as a positive compound control.
Screening for the affinity to FGFR1 was as follows: FGFR1 solutions with an appropriate concentration were taken, the positive compound SSR128129E (Sigma) at an appropriate concentration and sample solutions of the inventive polypeptide compounds, as freeze-dried powders, dissolved in protein buffers were then respectively added. After incubation at room temperature for 50 min, ultrafiltration and screening were carried out to obtain various treated solutions. An appropriate volume of acetonitrile was added to each treated solution to precipitate a protein, the solution was then centrifuged, and the supernatant was taken and analyzed by mass spectrometry.
Affinity screening conditions: One-dimensional liquid-phase conditions were: chromatographic column: PolyLC chromatographic column, mobile phase A: $KH_2PO4$, NaCl, pH 7.5, mobile phase B: acetonitrile, column temperature: 8°C, flow rate: 1 mL/min, and injection volume: appropriate; and two-dimensional liquid-phase conditions were: chromatographic column: BONUS RRHD, mobile phase A: 0.1% formic acid aqueous solution, mobile phase B: 0.1% formic acid in acetonitrile, column temperature: 60°C, flow rate: 0.3 mL/min, and injection volume: 40 uL. Gradient elution was used, i.e., 98% A (0-1 min), 98-10% A (1-6 min), 10% A (6-7.5 min), 10-98% A (7.5-8 min), and 98% A (8-9.5 min). Mass spectrometry conditions: Agilent 6530 Q-Tof, ESI ion source, voltage 3.5 KV, mass-to-charge ratio scanning range m/z = 200-3000, acquisition mode: positive ion scanning, ion source temperature: 350°C, solvent removal temperature: 300°C, and nitrogen flow rate: 8 L/min.
In the affinity screening results, Compounds 1 and 62-73 were found to have affinity for FGFR1.

Table 4. Screening results of polypeptide compounds with binding affinity to FGFR1

| No. | Cb/Ct range * |
| --- | --- |
| Positive | 4-5 |
| Compound 62 | 1.0-3 |
| Compound 63 | 1.0-3 |
| Compound 1 | 1.0-3 |
| Compound 64 | 1.0-3 |
| Compound 65 | 1.0-3 |
| Compound 66 | 1.0-3 |
| Compound 67 | 1.0-3 |
| Compound 68 | 1.0-3 |
| Compound 69 | 1.0-3 |
| Compound 70 | 1.0-3 |

(continued)

| No. | Cb/Ct range * |
|---|---|
| Compound 71 | 1.0-3 |
| Compound 72 | 1.0-3 |
| Compound 73 | 1.0-3 |

* The Cb/Ct ratio referred to the ratio of the amount of the lower-layer compound (mass spectrum signal intensity) to the amount of the upper-layer compound (mass spectrum signal intensity) during ultracentrifugation affinity screening. The greater the Cb/Ct ratio, the stronger the affinity.

**Example 3: Anti-ulcer effect of polypeptide compound of the present invention on ethanol-induced gastric ulcer model in mice**

**Experimental animals:** Six-week old C57BL/6JGPt male mice, weighing about 20 g/mouse, were selected, with 10 mice in each group, from GemPharmatech (Chengdu) Co., Ltd., animal license number: SCXK (Chuan) 2020-034.

**Experimental method:**

After the experimental animals were divided into groups, they were dosed according to Table 5 one day before the experiment. Among the groups, the model group was given 200 μl of pure water, and the other dose groups were given 200 μl of drug solution. After that, all the animals were fasted for 24 h with free access to water.

The next day, 1 h after administration, the mice in each group were given 9 μl/g of absolute ethanol by oral gavage for modeling. After 1 h, the animals were sacrificed by cervical dislocation, the gastric cardia was ligated, the pylorus was occluded, and the whole stomach was removed. 1 mL of 1% formaldehyde solution was injected into the gastric lumen via the glandular stomach, the cardia was ligated, and the stomach was taken out and immediately immersed in 1% formaldehyde solution. After soaking for 30 min, the stomach tissue was taken out and cut open along the greater curvature. The content of the stomach was rinsed off with normal saline, the injury of the gastric mucosa in the mice were observed and measured after being laid flat, and the ulcer index and ulcer inhibition rate were calculated; panoramic photos of the stomach were taken; and finally, the gastric tissue sample was immersed in formalin and fixed.

Calculation method for ulcer index: If the length of cord-like injury was greater than 1 mm, the length thereof was measured, with 1 point per millimeter; if the width thereof was greater than 1 mm, the score thereof was doubled according to the number of millimeters of the width; and if the length was less than 1 mm, a score of 0.5 was given, and the scores were added up to obtain the ulcer index of the animal.

$$\text{Ulcer inhibition rate } \% = (\text{ulcer index of model group - ulcer index of treatment group}) / \text{ulcer index of model group} * 100\%.$$

Method of data statistics: the ulcer index was expressed as mean $\pm$ standard deviation; the ulcer inhibition rate was calculated by using the median value and mean value of the ulcer index in each dosage group and simulation group; and the data were statistically analyzed by means of Excel, and the difference data of the gastric ulcer index were statistically analyzed by means of independent sample T test. Table 5 showed the results of the relative ulcer inhibition rates of the compounds of the present invention.

**Table 5. Anti-ulcer activity of the compound of the present invention in ethanol-induced model of mice after single administration**

| No. | Anti-ulcer activity * |
|---|---|
| 1 | 1.00 |
| 2 | 0.45 |
| 3 | 0.33 |
| 4 | 0.82 |
| 5 | 0.84 |

(continued)

| No. | Anti-ulcer activity * |
|---|---|
| 6 | ND |
| 7 | 1.69 |
| 8 | 0.89 |
| 9 | 0.59 |
| 10 | 0.56 |
| 11 | 1.08 |
| 12 | 0.68 |
| 13 | 1.02 |
| 14 | 0.49 |
| 15 | 0.72 |
| 16 | 0.98 |
| 17 | 0.74 |
| 18 | 0.91 |
| 19 | 0.64 |
| 20 | 0.56 |
| 21 | 0.87 |
| 22 | 0.81 |
| 23 | 0.70 |
| 24 | 0.62 |
| 25 | 0.36 |
| 26 | 0.56 |
| 27 | 0.51 |
| 28 | 0.67 |
| 29 | 1.00 |
| 30 | 0.33 |
| 31 | 0.50 |
| 32 | 0.52 |
| 33 | 0.31 |
| 34 | 0.91 |
| 35 | 0.57 |
| 36 | 0.07 |
| 37 | 0.22 |
| 38 | 1.38 |
| 39 | 0.83 |
| 40 | 0.18 |
| 41 | 0.92 |
| 42 | 0.84 |
| 43 | 0.30 |

(continued)

| No. | Anti-ulcer activity [*] |
| --- | --- |
| 44 | 0.16 |
| 45 | 0.50 |
| 46 | 0.47 |
| 47 | 0.61 |
| 48 | 1.19 |
| 49 | 0.48 |
| 50 | 0.09 |
| 51 | 0.62 |
| 52 | 0.67 |
| 53 | 0.44 |
| 54 | 0.59 |
| 55 | 0.20 |
| 56 | 0.91 |
| 57 | 1.12 |
| 58 | 0.52 |
| 59 | 0.48 |
| 60 | 1.03 |
| 61 | 0.52 |
| 62 | 0.89 |
| 63 | 1.99 |
| 64 | 0.61 |
| 65 | 1.29 |
| 66 | 0.52 |
| 67 | 0.24 |
| 68 | 0.85 |
| 69 | 1.25 |
| 70 | 0.39 |
| 71 | 0.77 |
| 72 | 1.65 |
| 73 | 1.46 |
| Teprenon e | 0.5 |

[*] Note:
1. The anti-ulcer effect of each compound was completed by several cohorts of experiments. For easy comparison, the anti-ulcer activity was expressed as the mean value of the relative ulcer inhibition rate (Compound 1 was used as a control group in each cohort of experiments), i.e., relative ulcer inhibition rate = (ulcer inhibition rate of test compound) / (ulcer inhibition rate of Compound 1).
2. ND represented no detection.

**Example 4: Pharmacodynamic study on the healing effect of the polypeptide compound of the present invention on acute mechanical skin injury in rats**

SPF grade SD rats, weighing 180-230 g, were raised in respective clean sterilized cages, with water, feed and padding replacement given regularly every day. The feeding temperature was kept at 22°C and the humidity was 55-65%. They were raised for one week for acclimatization. According to a random grouping method, the animals were divided into a model control group (normal saline), a Jinyintai control group (40 IU/cm$^2$, Shenzhen Watsin Genetech Ltd.) and a test polypeptide treatment group (40 μg/cm$^2$), with 6 animals in each group. After the rats were successfully anesthetized by intraperitoneal injection of 3% pentobarbital sodium, the hair within 1 cm around the edge of the wound was cut off, and the wound area was disinfected first with iodophor and then with 75% alcohol locally. A round full-thickness skin wound of 1.5 cm × 1.5 cm (i.e., 1.5 cm in diameter) was made near the neck at 4 cm down to the back from the middle of the connecting line of the ears, with the spine as the midline. It went deep into the muscle layer, forming an animal model of acute mechanical injury. After modeling, the rats were raised in individual cages with the wounds exposed. During dressing replacement, the rats were all first debrided with iodophor, and then, the wound was washed with sterile normal saline and wiped dry. Except the model group, the wounds of the rats in the other groups were locally coated with 40 μL of the corresponding drug solution once daily. On days 0, 5, 10 and 14 of administration, the wound images of the rats in each group were taken, and the wound area was calculated by means of an image analysis software (Image J). According to the formula, the wound healing rate was obtained.

The results of the study indicated that Polypeptide Compounds 68, 67 and 1 all had the trend of promoting wound healing. The results were shown in Table 6.

Table 6: Effects of promoting wound healing of the representative compounds of the present invention

| Group | 5 days | 10 days | 14 days |
|---|---|---|---|
| Model control group | 8.31±2.12 | 27.18±3.91 | 76.07±6.84 |
| Jinyintai control group | 25.35±5.24 | 59.93±5.88** | 86.99±5.42* |
| Compound 68 group | 29.26±6.46 | 60.97±2.87** | 88.42±4.79* |
| Compound 67 group | 30.40±3.35 | 61.42±5.73** | 90.87±3.94* |
| Compound 1 group | 31.68±3.48 | 63.20±3.87** | 91.43±2.87* |
| Note: * indicated that the sample group had $p < 0.05$, compared with the model group. | | | |

**Example 5: Therapeutic effect of Compound 1 on chronic atrophic gastritis model in mice**

Method: Chronic atrophic gastritis was induced by MNNG (N-methyl-N-nitro-N-nitrosoguanidine) combined with ranitidine. The mice were free to access to an aqueous solution containing MNNG (100 mg/ml), and at the same time, the mice were given ranitidine (8 mg/ml) aqueous solution at a dose of 150 mg/kg by gavage at a fixed time per day for 20 consecutive weeks. After 20 weeks of modeling, on the basis of drinking ordinary distilled water, the mice were given Compound 1 (5 mg/kg) daily by gavage. After 2 weeks of administration, the therapeutic effect of Compound 1 on chronic atrophic gastritis was observed.

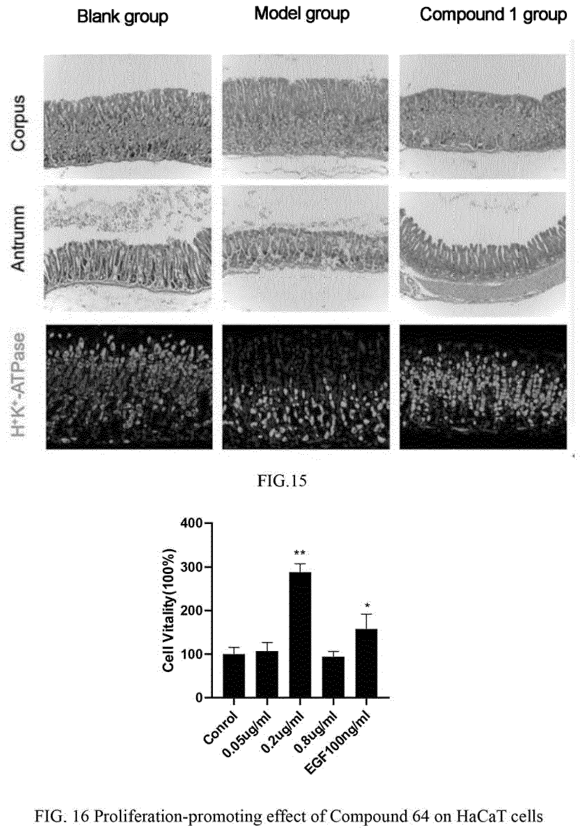

Results: The results of tissue staining showed that the gland structure in the corpus and antrum of model group was disordered, accompanied by a reduced number of parietal cells (H+-K+-ATPase positive) and decreased height of the mucosal epithelium in the gastric antrum. After 2 weeks of treatment with Compound 1, as compared with the model group, the structure of the gastric gland in the mice of the Compound 1 group was recovered to normal, the number of parietal cells increased significantly, and the height of the mucosa in the gastric antrum was substantially recovered to the normal state. The results were shown in Fig. 15. These results indicated that Compound 1 could promote the repair of chronic atrophic gastritis in mice.

**Example 6: Proliferation-promoting effect of the polypeptide compound of the present invention on HaCAT cells**

Experimental method: Immortalized human keratinocytes (HaCaT cells) was adjusted to a concentration of $1.0 \times 10^5$ to $5.0 \times 10^5$/mL for passaging, and they were cultured at 37°C and 5% $CO_2$ for 24-36 h for biological activity detection. The cells were digested with 0.25% trypsin for 5 min, 1640 whole blood culture medium, the volume of which was not less than 1 time of the volume of the trypsin, was added to stop digestion, a cell suspension was collected, centrifuged at 1000 RPM for 3 min, the supernatant was discarded, 2 mL of 1640 whole blood culture medium was added to suspend the cells, 20 uL of the cell suspension was taken and stained with AOPI, the concentration of cells in the suspension was then detected by a cell counter, the suspension was prepared to a concentration of $5\times10^4$/mL with 1640 culture medium containing serum at a concentration of 10% and inoculated into a 96-well cell culture plate, with 100 $\mu$L per well, i.e., 5000 cells/well, and the plate was cultured at 37°C and 5% $CO_2$ overnight.

After 24 h, the original culture medium was discarded, and 100 $\mu$L of polypeptide compound solutions with different concentrations prepared with 1640 culture medium containing serum at a concentration of 1% were added, such that the final concentrations of the polypeptide compounds to be tested were 0.2, 0.4 and 0.8 ug/mL, respectively. In addition, an EGF control group was prepared by adding 100 $\mu$L of a recombinant human epidermal growth factor (EGF) solution prepared with 1640 culture medium containing serum at a concentration of 1%, with the final concentration being 100 ng/mL. In the model control group, an equal volume of 1640 culture medium containing serum at a concentration of 1% was added. After culturing at 37°C and 5% $CO_2$ for 24 h, 48 h and 72 h, the proliferation of the HaCaT cell line was detected by means of CCK8 kit.

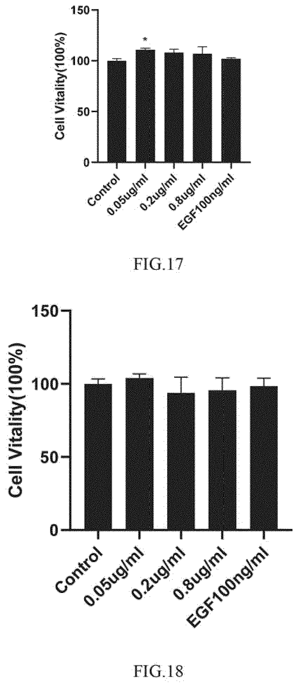

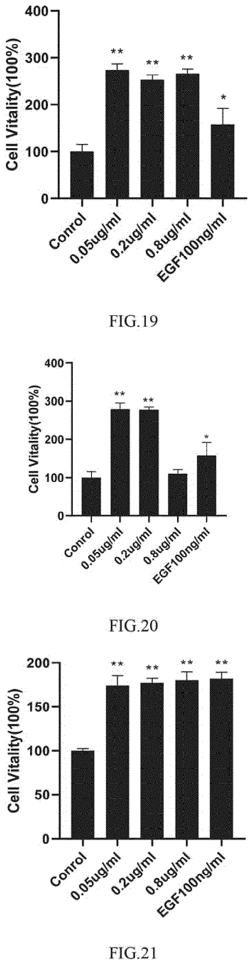

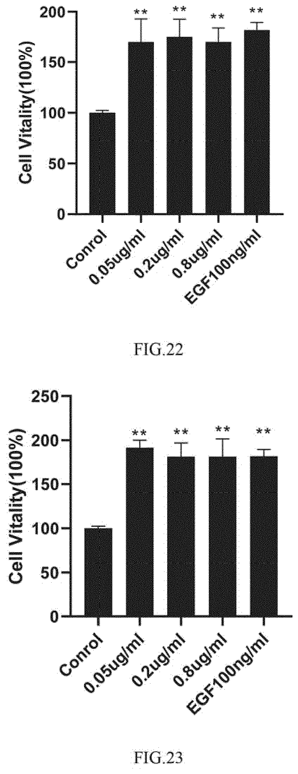

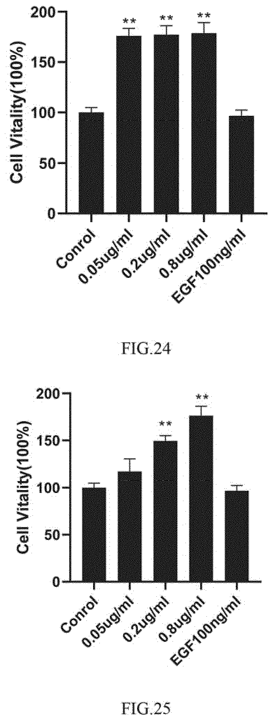

The experimental results were as shown in Figs. 16-27. Polypeptide Compounds 74, 73, 72, 71, 70, 69, 68, 67, 66, 65, 1, 64, and 63 had proliferation-promoting effects on HaCaT cells at a lower concentration (i.e., 0.2-0.8 $\mu$g/mL). (In the figures, * represented $P < 0.05$; and ** represented $P < 0.01$).

The results of the study indicated that most of the polypeptides H25 of the present invention had significant proliferation-promoting effects on HaCaT cells, and the effect was obviously superior to that of the EGF group (100 ng/mL).

**Example 7: Proliferation-promoting effect of the polypeptide compound of the present invention on HMEC-1 cells**

Human microvascular endothelial cells (HMEC-1 cells) were cultured in 10% serum culture medium at 37°C and 5% $CO_2$, the medium was replaced every 1-2 d, and the cell concentration was controlled to be $4\times10^6$ cells/mL for passage. Cells were collected, prepared to $5\times10^4$ cells/mL with complete culture medium, inoculated into a 96-well cell culture plate, with 100 uL per well, i.e., 5000 cells/well, and cultured at 37°C and 5% $CO_2$ until the cells adhered to the wall. The polypeptide compound of the present invention was prepared to test concentrations (0.05 ug/mL, 0.2 ug/mL, and 0.8 ug/mL) with 0% serum culture medium as experimental groups, a positive drug (300 ng/mL) was prepared in the same way and added as a positive control, and an equal amount of 0% serum drug-free culture medium was added to the control group. They were added to 5 duplicate wells, and the resultants were incubated at 37°C and 5% $CO_2$ for 48 h. Detection of the proliferation of cells using CCK-8: the old culture medium was removed, and a serum-free medium containing 10% CCK-8 was added to each well. After incubation in an incubator for 2 h, the absorbance at 450 nm was measured by a microplate reader.

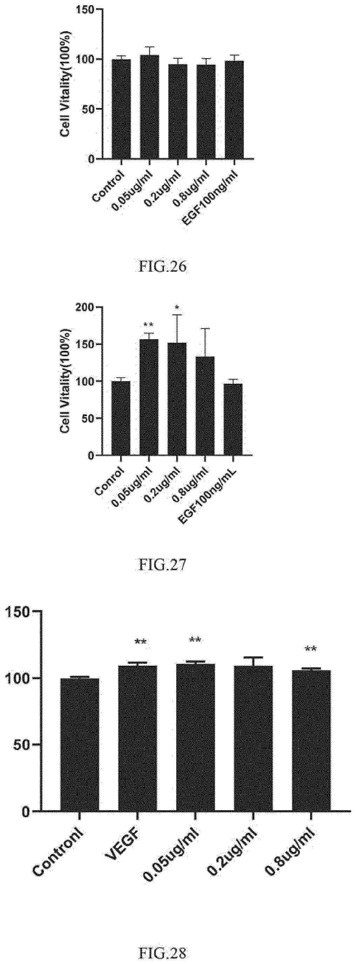

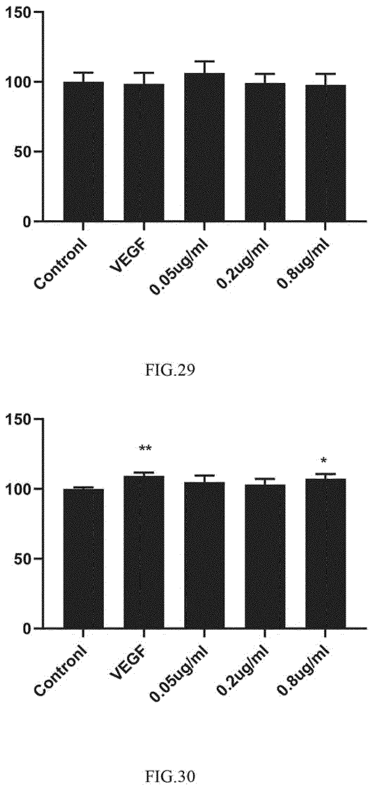

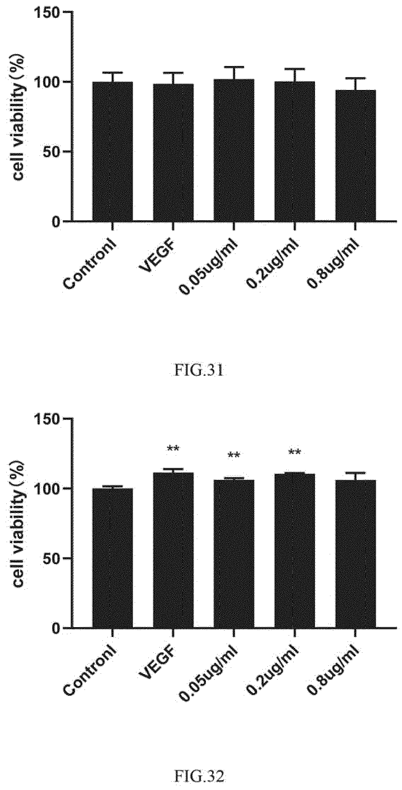

The experimental results were as shown in Figs. 28-32. Polypeptide Compounds 74, 72 and 1 all had significant proliferation promoting effects on HMEC-1 cells. In the figures, * represented promoting with $P < 0.05$; ** represented promoting with $P < 0.01$.

**Example 8: Effect of the polypeptides on the proliferation of RSC96 cells**

Glial cells (RSC96 cells) were adjusted to a concentration of $1.0\times10^5$ to $5.0\times10^5$/mL for passaging and cultured at 37°C and 5% $CO_2$ for 24-36 h for biological activity detection. The cells were digested by trypsin, collected, prepared to a concentration of $5\times10^4$/mL with a serum-free medium, inoculated into a 96-well cell culture plate, with 100 $\mu$L per well, i.e., 5000 cells/well, and cultured overnight at 37°C and 5% $CO_2$.

The polypeptide compound of the present invention was prepared to test concentrations (0.2 ug/ml, 0.4 ug/ml, and 0.8 ug/ml) with 0% serum culture medium as experimental groups, and an equal amount of 0% serum drug-free culture medium was added to the control group. They were added to 4 duplicate wells, and the resultants were incubated at 37°C and 5% $CO_2$ for 48 h.

Detection of the proliferation of cells using CCK-8: the old culture medium was removed, and a serum-free medium containing 10% CCK-8 was added to each well. After incubation in an incubator for 2 h, the absorbance at 450 nm was measured by a microplate reader.

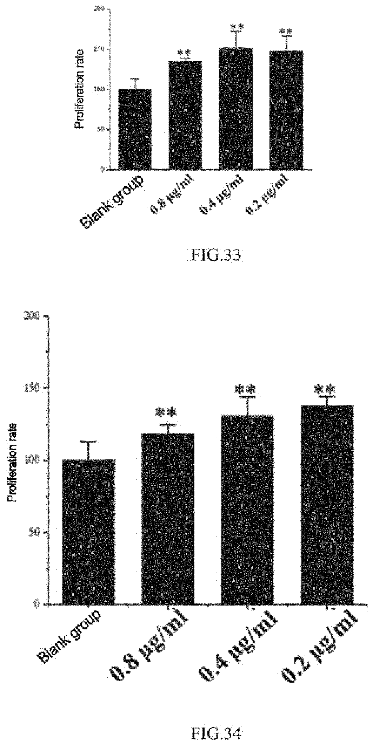

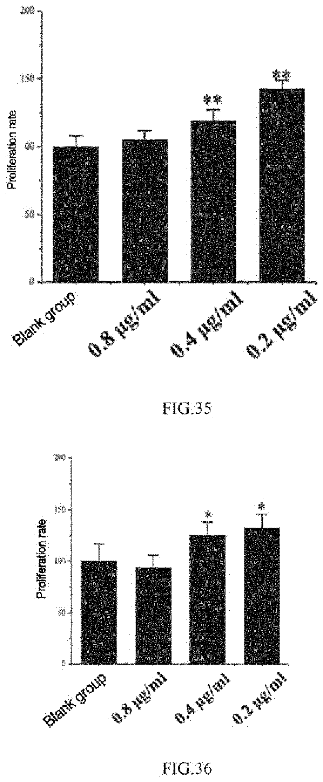

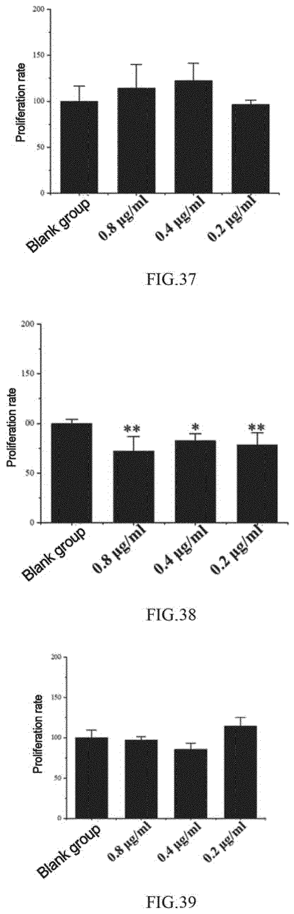

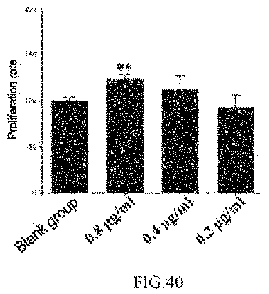

The experimental results were as shown in Figs. 33-40. Polypeptide Compounds 74, 68, 66, 1 and 64 all had significant proliferation promoting effects on RSC96 cells. In the figures, * represented $P < 0.05$, compared with the blank group; and ** represented $P < 0.01$, compared with the blank group.

Although the above examples are disclosed in the present invention, the embodiments of the present invention are not limited to the above examples, and any other changes, modifications, substitutions, combinations, and simplifications that do not depart from the present invention should be equivalent replacements and are included in the scope of protection of the present invention.

**Claims**

1. A compound of Formula (I) or a physiologically compatible salt thereof, wherein the compound of Formula (I) is as follows:

   H-$X_{aa1}$-$X_{aa2}$-$X_{aa3}$-$X_{aa4}$-$X_{aa5}$-$X_{aa6}$-$X_{aa7}$-$X_{aa8}$-$X_{aa9}$-$X_{aa10}$-$X_{aa11}$-$X_{aa12}$-$X_{aa13}$-$X_{aa14}$-$X_{aa15}$-$X_{aa16}$-$X_{aa17}$-$X_{aa18}$-$X_{aa19}$-OH     **(I),**

   wherein

   $X_{aa1}$ is Pro or absent;
   $X_{aa2}$ is Ala or absent;
   $X_{aa3}$ is Ser, Ala, Thr or absent;
   Xaa4 is Met, Met(O), Val, Ala, Leu, Ile, Thr, Glu, Arg or absent;
   $X_{aa5}$ is Gln, Ala, Glu, Pro, Lys or absent;
   $X_{aa6}$ is Ala, Ser, Val, Asp or absent;
   $X_{aa7}$ is Ser, Ala, Thr, Pro, Val or absent;
   $X_{aa8}$ is Leu, Ala, Val, Tyr or absent;
   $X_{aa9}$ is Glu, Gln, Ala, Leu, Asp or absent;
   $X_{aa10}$ is Ala, Ser or absent;
   $X_{aa11}$ is Glu, Gln, Ala, Leu, Asp or absent;
   $X_{aa12}$ is Ala, Ser or absent;
   $X_{aa13}$ is Lys, Ala, Arg, His or absent;
   $X_{aa14}$ is Gly, Ala, Pro or absent;
   $X_{aa15}$ is Lys, Arg, Ala, His or absent;
   $X_{aa16}$ is Ala, Ser or absent;
   $X_{aa17}$ is Glu, Gln, Asp, Asn, Ala, Lys or absent;
   $X_{aa18}$ is Asp, Glu, Leu, Ala, Gln or absent; and
   $X_{aa19}$ is Asp, Glu or absent; and
   provided that at least four of $X_{aa1}$, $X_{aa2}$, $X_{aa3}$, Xaa4, $X_{aa5}$, $X_{aa6}$, $X_{aa7}$, $X_{aa8}$, $X_{aa9}$, $X_{aa10}$, $X_{aa11}$, $X_{aa12}$, $X_{aa13}$, $X_{aa14}$, $X_{aa15}$, $X_{aa16}$, $X_{aa17}$, $X_{aa18}$, and $X_{aa19}$ are not absent.

2. The compound or physiologically compatible salt thereof according to claim 1, wherein $X_{aa1}$ and $X_{aa2}$ are both absent.

3. The compound or physiologically compatible salt thereof according to claim 1 or 2, wherein $X_{aa3}$ is Ser or absent, preferably Ser.

4. The compound or physiologically compatible salt thereof according to any one of claims 1-3, wherein $X_{aa4}$ is Met or Met(O) or absent, preferably Met or Met(O).

5. The compound or physiologically compatible salt thereof according to any one of claims 1-4, wherein $X_{aa3}$-$X_{aa4}$ is Ser-Met, Ser-Met(O), Ser-Val, Ser-Ala, Ser-Leu, Ser-Ile, Thr -Met or absent, preferably Ser-Met, Ser-Met(O) or absent, more preferably Ser-Met.

6. The compound or physiologically compatible salt thereof according to any one of claims 1-5, wherein $X_{aa5}$ is Gln or absent, preferably Gln.

7. The compound or physiologically compatible salt thereof according to any one of claims 1-6, wherein $X_{aa6}$ is Ala or absent, preferably Ala.

8. The compound or physiologically compatible salt thereof according to any one of claims 1-7, wherein $X_{aa7}$ is Ser or absent, preferably Ser.

9. The compound or physiologically compatible salt thereof according to any one of claims 1-8, wherein $X_{aa8}$ is Leu or absent, preferably Leu.

10. The compound or physiologically compatible salt thereof according to any one of claims 1-9, wherein

$X_{aa5}$-$X_{aa6}$-$X_{aa7}$-$X_{aa8}$ is Gln-Ala-Ser-Leu, Gln-Ala-Ser-Ala, Ala-Ala-Ser-Leu, Gln-Ala-Ala-Leu, Glu-Ala-Ser-Leu, Gln-Ala-Thr-Leu, Gln-Ala-Ser-Val, Ala-Ser-Thr-Leu, Pro-Val-Pro-Leu, Lys-Asp-Val-Tyr or absent, preferably Gln-Ala-Ser-Leu.

11. The compound or physiologically compatible salt thereof according to any one of claims 1-10, wherein $X_{aa9}$ is Glu or Gln.

12. The compound or physiologically compatible salt thereof according to any one of claims 1-11, wherein $X_{aa10}$ is Ala.

13. The compound or physiologically compatible salt thereof according to any one of claims 1-12, wherein $X_{aa11}$ is Glu, Gln or Ala.

14. The compound or physiologically compatible salt thereof according to any one of claims 1-13, wherein $X_{aa12}$ is Ala.

15. The compound or physiologically compatible salt thereof according to any one of claims 1-14, wherein Xaal3 is Lys.

16. The compound or physiologically compatible salt thereof according to any one of claims 1-15, wherein $X_{aa9}$-$X_{aa10}$-$X_{aa11}$-$X_{aa12}$ is Glu-Ala-Glu-Ala, Gln-Ala-Gln-Ala, Ala-Ala-Glu-Ala, Glu-Ala-Ala-Ala, Leu-Ala-Glu-Ala, Glu-Ala-Leu-Ala, Gln-Ala-Glu-Ala, Glu-Ala-Gln-Ala, Asp-Ala-Glu-Ala, Glu-Ala-Asp-Ala, Glu-Ser-Glu-Ala, Glu-Ala-Glu-Ser or absent.

17. The compound or physiologically compatible salt thereof according to any one of claims 1-16, wherein $X_{aa9}$-$X_{aa10}$-$X_{aa11}$-$X_{aa12}$-$X_{aa13}$ is Glu-Ala-Glu-Ala-Lys, Gln-Ala-Gln-Ala-Lys, Glu-Ala-Glu-Ala-Ala, Ala-Ala-Glu-Ala-Lys, Glu-Ala-Ala-Ala-Lys, Glu-Ala-Glu-Ala-Arg, Glu-Ala-Glu-Ala-His, Leu-Ala-Glu-Ala-Lys, Glu-Ala-Leu-Ala-Lys, Gln-Ala-Glu-Ala-Lys, Glu-Ala-Gln-Ala-Lys, Asp-Ala-Glu-Ala-Lys, Glu-Ala-Asp-Ala-Lys, Glu-Ser-Glu-Ala-Lys, Glu-Ala-Glu-Ser-Lys, Glu-Ala-Glu-Ala or absent, preferably Glu-Ala-Glu-Ala-Lys.

18. The compound or physiologically compatible salt thereof according to any one of claims 1-17, wherein Xaal4 is Gly.

19. The compound or physiologically compatible salt thereof according to any one of claims 1-18, wherein Xaal5 is Lys.

20. The compound or physiologically compatible salt thereof according to any one of claims 1-19, wherein $X_{aa14}$-$X_{aa15}$ is Gly-Lys, Gly-Arg, Gly-Ala, Gly-His, Ala-Lys, Pro-Lys or absent, preferably Gly-Lys.

21. The compound or physiologically compatible salt thereof according to any one of claims 1-19, wherein $X_{aa16}$ is Ala.

22. The compound or physiologically compatible salt thereof according to any one of claims 1-21, wherein $X_{aa17}$ is Glu.

23. The compound or physiologically compatible salt thereof according to any one of claims 1-22, wherein $X_{aa16}$-$X_{aa17}$ is Ala-Glu, Ala-Gln, Ala-Asp, Ala-Asn, Ala-Ala, Ala-Lys, Ser-Glu or absent.

24. The compound or physiologically compatible salt thereof according to any one of claims 1-23, wherein $X_{aa18}$-$X_{aa19}$ is Asp, Glu, Leu, Ala, Gln-Asp, Gln-Glu or absent.

25. The compound or physiologically compatible salt thereof according to claim 1, wherein the compound is selected from:

Ser-Met-Gln-Ala-Ser-Leu-Glu-Ala-Glu-Ala-Lys-Gly-Lys-Ala-Glu (Compound 1);
Gln-Ala-Ser-Leu-Glu-Ala-Glu-Ala-Lys-Gly-Lys-Ala-Glu (Compound 2);
Ser-Met-Gln-Ala-Ser-Leu-Glu-Ala-Glu-Ala-Lys-Gly-Lys (Compound 3);
Gln-Ala-Ser-Leu-Glu-Ala-Glu-Ala-Lys-Gly-Lys (Compound 4);
Ser-Met-Gln-Ala-Ser-Leu-Glu-Ala-Glu-Ala-Lys (Compound 5);
Gln-Ala-Ser-Leu-Glu-Ala-Glu-Ala-Lys (Compound 6);
Glu-Ala-Glu-Ala-Lys-Gly-Lys-Ala-Glu (Compound 7);
Ser-Met-Gln-Ala-Ser-Leu (Compound 8);
Gln-Ala-Ser-Leu (Compound 9);
Gly-Lys-Ala-Glu (Compound 10);
Ser-Met(O)-Gln-Ala-Ser-Leu-Glu-Ala-Glu-Ala-Lys-Gly-Lys-Ala-Glu (Compound 11);
Ser-Val-Gln-Ala-Ser-Leu-Glu-Ala-Glu-Ala-Lys-Gly-Lys-Ala-Glu (Compound 12);

Ser-Met-Gln-Ala-Ser-Leu-Glu-Ala-Glu-Ala-Lys-Gly-Lys-Ala-Gln (Compound 13);
Ser-Met-Gln-Ala-Ser-Leu-Gln-Ala-Gln-Ala-Lys-Gly-Lys-Ala-Glu (Compound 14);
Ser-Met-Gln-Ala-Ser-Ala-Glu-Ala-Glu-Ala-Lys-Gly-Lys-Ala-Glu (Compound 15);
Ser-Ala-Gln-Ala-Ser-Leu-Glu-Ala-Glu-Ala-Lys-Gly-Lys-Ala-Glu (Compound 16);
Ser-Leu-Gln-Ala-Ser-Leu-Glu-Ala-Glu-Ala-Lys-Gly-Lys-Ala-Glu (Compound 17);
Ser-Met-Gln-Ala-Ser-Leu-Glu-Ala-Glu-Ala-Ala-Gly-Lys-Ala-Glu (Compound 18);
Ser-Met-Gln-Ala-Ser-Leu-Ala-Ala-Glu-Ala-Lys-Gly-Lys-Ala-Glu (Compound 19);
Ser-Met-Gln-Ala-Ser-Leu-Glu-Ala-Ala-Ala-Lys-Gly-Lys-Ala-Glu (Compound 20);
Ser-Met-Ala-Ala-Ser-Leu-Glu-Ala-Glu-Ala-Lys-Gly-Lys-Ala-Glu (Compound 21);
Ser-Ile-Gln-Ala-Ser-Leu-Glu-Ala-Glu-Ala-Lys-Gly-Lys-Ala-Glu (Compound 22);
Ser-Met-Gln-Ala-Ser-Leu-Glu-Ala-Glu-Ala-Lys-Gly-Lys-Ala-Asp (Compound 23);
Ser-Met-Gln-Ala-Ser-Leu-Glu-Ala-Glu-Ala-Lys-Gly-Lys-Ala-Asn (Compound 24);
Ser-Met-Gln-Ala-Ser-Leu-Glu-Ala-Glu-Ala-Lys-Gly-Arg-Ala-Glu (Compound 25);
Ser-Met-Gln-Ala-Ser-Leu-Glu-Ala-Glu-Ala-Lys-Gly-Ala-Ala-Glu (Compound 26);
Ser-Met-Gln-Ala-Ser-Leu-Glu-Ala-Glu-Ala-Lys-Gly-Lys-Ala-Ala (Compound 27);
Ser-Met-Gln-Ala-Ala-Leu-Glu-Ala-Glu-Ala-Lys-Gly-Lys-Ala-Glu (Compound 28);
Ala-Met-Gln-Ala-Ser-Leu-Glu-Ala-Glu-Ala-Lys-Gly-Lys-Ala-Glu (Compound 29);
Ser-Met-Gln-Ala-Ser-Leu-Glu-Ala-Glu-Ala-Arg-Gly-Lys-Ala-Glu (Compound 30);
Ser-Met-Gln-Ala-Ser-Leu-Glu-Ala-Glu-Ala-Lys-Gly-His-Ala-Glu (Compound 31);
Ser-Met-Gln-Ala-Ser-Leu-Glu-Ala-Glu-Ala-His-Gly-Lys-Ala-Glu (Compound 32);
Ser-Met-Gln-Ala-Ser-Leu-Leu-Ala-Glu-Ala-Lys-Gly-Lys-Ala-Glu (Compound 33);
Ser-Met-Gln-Ala-Ser-Leu-Glu-Ala-Leu-Ala-Lys-Gly-Lys-Ala-Glu (Compound 34);
Ser-Met-Gln-Ala-Ser-Leu-Gln-Ala-Glu-Ala-Lys-Gly-Lys-Ala-Glu (Compound 35);
Ser-Met-Gln-Ala-Ser-Leu-Glu-Ala-Gln-Ala-Lys-Gly-Lys-Ala-Glu (Compound 36);
Ser-Met-Glu-Ala-Ser-Leu-Glu-Ala-Glu-Ala-Lys-Gly-Lys-Ala-Glu (Compound 37);
Ser-Met-Gln-Ala-Ser-Leu-Asp-Ala-Glu-Ala-Lys-Gly-Lys-Ala-Glu (Compound 38);
Ser-Met-Gln-Ala-Ser-Leu-Glu-Ala-Asp-Ala-Lys-Gly-Lys-Ala-Glu (Compound 39);
Thr-Met-Gln-Ala-Ser-Leu-Glu-Ala-Glu-Ala-Lys-Gly-Lys-Ala-Glu (Compound 40);
Ser-Met-Gln-Ala-Thr-Leu-Glu-Ala-Glu-Ala-Lys-Gly-Lys-Ala-Glu (Compound 41);
Ser-Met-Gln-Ala-Ser-Val-Glu-Ala-Glu-Ala-Lys-Gly-Lys-Ala-Glu (Compound 42);
Ser-Met-Gln-Ala-Ser-Leu-Glu-Ser-Glu-Ala-Lys-Gly-Lys-Ala-Glu (Compound 43);
Ser-Met-Gln-Ala-Ser-Leu-Glu-Ala-Glu-Ser-Lys-Gly-Lys-Ala-Glu (Compound 44);
Glu-Ala-Glu-Ala (Compound 45);
Glu-Ala-Glu-Ala-Lys-Gly-Lys (Compound 46);
Thr-Ala-Ser-Thr-Leu-Glu-Ala-Glu-Ala-Lys-Gly-Lys-Ala-Glu (Compound 47);
Ser-Thr-Gln-Ala-Ser-Leu-Glu-Ala-Glu-Ala-Lys-Gly-Lys-Ala-Glu (Compound 48);
Ser-Met-Gln-Ala-Ser-Leu-Glu-Ala-Glu-Ala-Lys-Gly-Lys-Ala-Gln-Asp (Compound 49);
Ser-Met-Gln-Ala-Ser-Leu-Glu-Ala-Glu-Ala-Lys-Gly-Lys-Ala-Gln-Glu (Compound 50);
Ser-Met-Gln-Ala-Ser-Leu-Glu-Ala-Glu-Ala-Lys-Gly-Lys-Ala-Glu-Leu (Compound 51);
Ser-Met-Gln-Ala-Ser-Leu-Glu-Ala-Glu-Ala-Lys-Gly-Lys-Ala-Glu-Ala (Compound 52);
Ser-Met-Gln-Ala-Ser-Leu-Glu-Ala-Glu-Ala-Lys-Gly-Lys-Ala-Lys-Gln-Asp (Compound 53);
Ala-Glu-Pro-Val-Pro-Leu-Glu-Ala-Glu-Ala-Lys-Gly-Lys-Ala-Glu (Compound 54);
Pro-Ala-Ala-Glu-Pro-Val-Pro-Leu-Glu-Ala-Glu-Ala-Lys-Gly-Lys-Ala-Glu (Compound 55);
Ser-Met-Gln-Ala-Ser-Leu-Glu-Ala-Glu-Ala-Lys-Ala-Lys-Ala-Glu (Compound 56);
Ser-Met-Gln-Ala-Ser-Leu-Glu-Ala-Glu-Ala-Lys-Pro-Lys-Ala-Glu (Compound 57);
Ser-Met-Gln-Ala-Ser-Leu-Glu-Ala-Glu-Ala-Lys-Gly-Lys-Ala-Lys-Gln-Glu (Compound 58);
Arg-Lys-Asp-Val-Tyr-Glu-Ala-Glu-Ala-Lys-Gly-Lys-Ala-Glu (Compound 59);
Ser-Met-Gln-Ala-Ser-Leu-Glu-Ala-Glu-Ala-Lys-Gly-Lys-Ser-Glu (Compound 60); or
Glu-Ala-Glu-Ala-Ala-Gly-Lys-Ala-Glu (Compound 61).

**26.** A compound or a physiologically compatible salt thereof, wherein the compound is:

Phe-Asp-Ala-Leu-Lys-Gln-Gln-Phe-Gln-Ala-Phe-Gln-Leu-Glu (Compound 62);
His-Cys-Leu-Ala-Gly-Leu-Lys-Lys-Asp-Leu-Glu-Asp-Leu-Glu (Compound 63);
Glu-Ile-Asn-Gln-Leu-Glu-Leu-Ile-Lys-Gln-Ala-Ser-Ile (Compound 64);
Ala-Ala-Arg-Leu-Ala-Asp-Glu-Leu-Arg-Ala-Glu (Compound 65);
Glu-Thr-Leu-Gln-Arg-Lys-Asn-Lys-Glu (Compound 66);
Val-Asp-Ala-Ala-Val-Leu-Glu-Lys-Leu-Glu (Compound 67);

Ala-Ala-Val-Leu-Asp-Lys-Leu-Glu (Compound 68);
Ala-Ala-Val-Leu-Glu-Lys-Leu-Glu (Compound 69);
Lys-Phe-Tyr-Ser-Gln-Ser-Thr-Ala-Ser-Ser-Ser-Tyr-Ala-Tyr-Pro-Ser-His-Phe-Gly-Pro-Ala-Gly-Phe-Ser-Gly-Ser-His-Ser (Compound 70);
Val-Asp-Ala-Ala-Val-Ile-Glu-Lys-Ile-Glu (Compound 71);
Val-Asp-Ala-Ala-Met-Val-Leu-Leu-Thr-Arg (Compound 72); or
Val-Asp-Ala-Ala-Val-Leu-Met-Leu-Arg-Thr (Compound 73).

27. Use of the compound or physiologically compatible salt thereof according to any one of claims 1-26 in the preparation of a medicament for repairing skin wound or mucosal damage.

28. The use according to claim 27, wherein the mucosal damage is mucosal damage in the cavity of the digestive system or respiratory system.

29. The use according to claim 28, wherein the mucosal damage of the digestive system is related to oral, esophageal, and gastrointestinal diseases, and the oral diseases include oral ulcer, stomatitis, gingivitis, and periodontitis; the esophageal diseases include esophagitis and esophageal ulcer; the gastrointestinal diseases include chronic gastritis, chronic atrophic gastritis, acute gastritis, gastroduodenal ulcer, functional gastrointestinal diseases, dyspepsia, precancerous lesions, digestive system tumors, gastrointestinal bleeding, gastroesophageal reflux disease, acute and chronic enteritis, ulcerative colitis, Crohn's disease, and mucosal damages caused by radiotherapy and/or chemotherapy; and the skin wound is related to diseases of epidermal inflammation, mechanical and surgical wound, burns and scalds, ulcers, fistulas, bedsores, and skin injuries caused by radiotherapy and/or chemotherapy.

30. The use according to claim 29, wherein the mucosal damage of the digestive system is mucosal damage caused by an irritant substance or a drug or by a stress state.

31. Use of the compound or physiologically compatible salt thereof according to any one of claims 1-26 in the preparation of a medicament for preventing, alleviating or treating a gastrointestinal disease or eliminating inflammatory edema.

32. A method for blocking or activating FGF/FGFR1 signaling, said method comprising administering the compound or physiologically compatible salt thereof according to any one of claims 1-26 to a subject.

33. A method for treating a disease associated with FGFR target, said method comprising administering the compound or physiologically compatible salt thereof according to any one of claims 1-26 to a subject.

34. A pharmaceutical composition, food composition, health care or cosmetic composition, or commodity composition, said composition comprising the compound or physiologically compatible salt thereof according to any one of claims 1-26 and a physiologically acceptable carrier.

FIG.1

FIG.2

FIG.3

FIG.4

FIG.5

FIG.6

FIG.7

FIG.8

FIG.9

FIG.10

FIG.11

FIG.12

FIG.13

FIG.14

FIG.15

FIG. 16 Proliferation-promoting effect of Compound 64 on HaCaT cells

FIG.17

FIG.18

FIG.19

FIG.20

FIG.21

FIG.22

FIG.23

FIG.24

FIG.25

FIG.26

FIG.27

FIG.28

FIG.29

FIG.30

FIG.31

FIG.32

FIG.33

FIG.34

FIG.35

FIG.36

FIG.37

FIG.38

FIG.39

FIG.40

## INTERNATIONAL SEARCH REPORT

| International application No. |
|---|
| **PCT/CN2021/142838** |

**A.　CLASSIFICATION OF SUBJECT MATTER**

C07K 7/08(2006.01)i;　C07K 7/06(2006.01)i;　A61K 38/10(2006.01)i;　A61K 38/08(2019.01)i;　A61P 1/04(2006.01)i;　A61P 17/02(2006.01)i

According to International Patent Classification (IPC) or to both national classification and IPC

**B.　FIELDS SEARCHED**

Minimum documentation searched (classification system followed by classification symbols)

　　C07K; A61K; A61P

Documentation searched other than minimum documentation to the extent that such documents are included in the fields searched

Electronic data base consulted during the international search (name of data base and, where practicable, search terms used)

　　CNABS, CNTXT, VEN, USTXT, WOTXT, EPTXT, CNKI, 万方, WANFANG, Pubmed, ISI web of knowledge, STN, Genbank, EMBL: SEQ ID NOs. 1-73, 皮肤, 黏膜, 粘膜, 伤口, 溃疡, 修复, 治疗, 愈合, skin, mucous, ulcer, wound, heal, therapy, treat, FGF, 成纤维细胞生长因子, fibroblast growth factor, 四川好医生攀西药业有限责任公司, 耿福能

**C.　DOCUMENTS CONSIDERED TO BE RELEVANT**

| Category* | Citation of document, with indication, where appropriate, of the relevant passages | Relevant to claim No. |
|---|---|---|
| X | WO 0196364 A2 (IMPERIAL COLLEGE INNOVATIONS LIMITED et al.) 20 December 2001 (2001-12-20)<br>　　description, page 2, paragraph 2, page 6, paragraph 1 to page 8, last paragraph, page 15, paragraph 4, and page 19, paragraph 2 | 1-24, 27-34 |
| X | WO 2007002469 A2 (BHATNAGAR, R. S.) 04 January 2007 (2007-01-04)<br>　　description, paragraphs 3, 4, 94-104, and 123-129 | 1-24, 27-31, 34 |
| X | WO 2014200910 A2 (IOGENETICS, LLC.) 18 December 2014 (2014-12-18)<br>　　claims 59, 66, and 75-78, and description, page 21, paragraphs 2 and 3, and a sequence table | 1-25, 34 |
| X | CN 1405298 A (HAINAN HAILIANG BIOLOG HIGH-TECHNOLOGY CO., LTD.) 26 March 2003 (2003-03-26)<br>　　claim 1 | 1-16, 20, 23-25 |
| X | CN 109206482 A (FUJIAN LANHAO BIOLOGICAL TECHNOLOGY CO., LTD.) 15 January 2019 (2019-01-15)<br>　　description, paragraphs 5-13 and 25 | 1-24, 27-34 |

☑ Further documents are listed in the continuation of Box C.　　☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **11 March 2022** | **19 April 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| | International application No. |
|---|---|
| | **PCT/CN2021/142838** |

### C. DOCUMENTS CONSIDERED TO BE RELEVANT

| Category* | Citation of document, with indication, where appropriate, of the relevant passages | Relevant to claim No. |
|---|---|---|
| X | 宗宪磊 等 (SONG, Xianlei et al.). "应用噬菌体随机七肽库筛选hFGF-7模拟肽 (Non-official translation: Screening of a hFGF-7 Mimetic Peptide using a Bacteriophage Random Heptapeptide Library)" <br> *2009中华医学会第六次全国医学美容与美容学术年会 (Non-official translation: 6th National Medical Cosmetology and Cosmetology Academic Annual Conference of 2009 Chinese Medical Association),* 31 December 2009 (2009-12-31), <br> pages 521-527 | 1-24, 27-34 |
| X | 宗宪磊 等 (SONG, Xianlei et al.). "应用噬菌体随机七肽库筛选人角质细胞生长因子模拟肽 (Screening Human Keratinocyte Growth Factor Mimic Peptide with Ph.D.-7TM Phage Display Peptide Library)" <br> *中国修复重建外科杂志 (Chinese Journal of Reparative and Reconstructive Surgery),* Vol. 23, No. 2, 28 February 2009 (2009-02-28), <br> pages 183-187 | 1-24, 27-34 |
| A | CN 107973838 A (JINAN UNIVERSITY) 01 May 2018 (2018-05-01) <br> entire document | 1-34 |
| A | CN 106977586 A (JINAN UNIVERSITY) 25 July 2017 (2017-07-25) <br> entire document | 1-34 |
| A | CN 102453079 A (LI, Xiaokun) 16 May 2012 (2012-05-16) <br> entire document | 1-34 |
| A | XIE, Yangli et al. "FGF/FGFR Signaling in Health and Disease" <br> *Signal Transduction and Targeted Therapy,* 02 September 2020 (2020-09-02), <br> Article number: 181, and pages 1-38 | 1-34 |

Form PCT/ISA/210 (second sheet) (January 2015)

**INTERNATIONAL SEARCH REPORT**

International application No.

**PCT/CN2021/142838**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

## INTERNATIONAL SEARCH REPORT

| International application No. |
|---|
| **PCT/CN2021/142838** |

**Box No. II        Observations where certain claims were found unsearchable (Continuation of item 2 of first sheet)**

This international search report has not been established in respect of certain claims under Article 17(2)(a) for the following reasons:

1. ☑  Claims Nos.: **32-33**
    because they relate to subject matter not required to be searched by this Authority, namely:

    [1]   Claim 32 relates to a method for blocking or activating FGF/FGFR1 signal transmission. Claim 33
          relates to a method for treating diseases associated with an FGFR target. That is, claims 32 and 33
          relate to the subject matter as defined in PCT Rule 39.1 that does not warrant a search conducted
          by the international searching authority: (4) a method for the treatment of a human or animal body
          by surgery or therapy, and a diagnostic method implemented on the human or animal body. An
          international search is formed on the basis of a use of the compound according to any one of claims
          1-26 or a physiologically compatible salt thereof in the preparation of drugs for blocking or activating
          FGF/FGFR1 signal transmission or for treating diseases associated with an FGFR target.

2. ☐  Claims Nos.:
    because they relate to parts of the international application that do not comply with the prescribed requirements to such an
    extent that no meaningful international search can be carried out, specifically:

3. ☐  Claims Nos.:
    because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2021/142838**

| Patent document cited in search report | | | Publication date (day/month/year) | Patent family member(s) | | | Publication date (day/month/year) |
|---|---|---|---|---|---|---|---|
| WO | 0196364 | A2 | 20 December 2001 | US | 2008096796 | A1 | 24 April 2008 |
| | | | | EP | 1294744 | A2 | 26 March 2003 |
| | | | | WO | 0196364 | A3 | 30 May 2002 |
| | | | | US | 2004102370 | A1 | 27 May 2004 |
| | | | | US | 7304129 | B2 | 04 December 2007 |
| | | | | GB | 0014870 | D0 | 09 August 2000 |
| WO | 2007002469 | A2 | 04 January 2007 | WO | 2007002469 | A3 | 21 June 2007 |
| | | | | US | 2006293228 | A1 | 28 December 2006 |
| WO | 2014200910 | A2 | 18 December 2014 | WO | 2014200910 | A3 | 26 February 2015 |
| | | | | US | 2016132631 | A1 | 12 May 2016 |
| CN | 1405298 | A | 26 March 2003 | CN | 1238492 | C | 25 January 2006 |
| CN | 109206482 | A | 15 January 2019 | None | | | |
| CN | 107973838 | A | 01 May 2018 | None | | | |
| CN | 106977586 | A | 25 July 2017 | None | | | |
| CN | 102453079 | A | 16 May 2012 | CN | 102453079 | B | 08 May 2013 |

Form PCT/ISA/210 (patent family annex) (January 2015)